# FASCICULE DE BREVET EUROPEEN

(11) **EP 3 353 184 B1**
(45) Date de publication et mention de la délivrance du brevet: **24.03.2021**
(21) Numéro de dépôt: 16785211.0
(22) Date de dépôt: 23.09.2016
(51) Int. Cl.: C07F 9/24, A61K 9/127, A61K 47/00

(54) **NOUVEAUX LIPIDES AMPHIPHILES RAMIFIÉS**
NEUARTIGE VERZWEIGTE AMPHIPHILE LIPIDE
NOVEL BRANCHED AMPHIPHILIC LIPIDS

(30) Priorité: 25.09.2015 FR 1559066
(43) Date de publication de la demande: 01.08.2018
(73) Titulaire: Université de Bretagne Occidentale, 29238 Brest (FR); INSERM (Institut National de la Santé et de la Recherche Médicale), 75654 Paris Cedex 13 (FR); Centre Hospitalier Régional et Universitaire de Brest, 29609 Brest Cedex (FR)
(72) Inventeur: JAFFRÈS, Paul-Alain, 29820 Bohars (FR); COUTHON-COURVÈS, Hélène, 29820 Bohars (FR); AFONSO, Damien, 29200 Brest (FR); MONTIER, Tristan, 29200 Brest (FR); LE GALL, Tony, 29200 Brest (FR)
(74) Mandataire: Icosa
(86) Numéro de dépôt international: PCT/FR2016/052410
(87) Numéro de publication internationale: WO 2017/051129

(56) Documents cités:
- MATTIAS F LINDBERG ET AL: "The gene transfection properties of a lipophosphoramidate derivative with two phytanyl chains", BIOMATERIALS, ELSEVIER SCIENCE PUBLISHERS BV., BARKING, GB, vol. 33, no. 26, 8 mai 2012 (2012-05-08), pages 6240-6253, XP028500390, ISSN: 0142-9612, DOI: 10.1016/J.BIOMATERIALS.2012.05.014 [extrait le 2012-05-17]

## Description

### DOMAINE DE L'INVENTION

La présente invention concerne de nouveaux lipides amphiphiles ramifiés. En particulier, la présente invention concerne de nouveaux lipides amphiphiles ramifiés de formule générale (II). La présente invention propose également un procédé de synthèse pour lesdits composés de formule (II), à partir des composés amphiphiles insaturés de formule générale (I). L'invention concerne également l'utilisation des composés de formule générale (II) et des lipoplexes obtenus par formulation des composés de formule générale (II) pour des applications, notamment la transfection, dans lesquelles des propriétés de fusion améliorées sont recherchées.

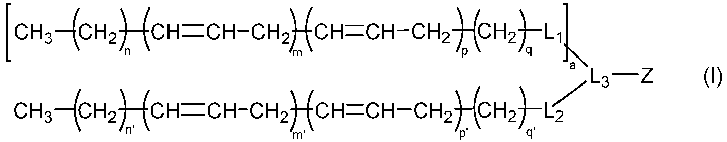

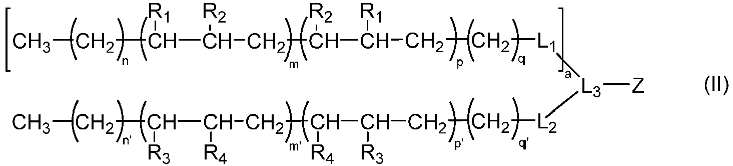

### ÉTAT DE LA TECHNIQUE

Les lipides amphiphiles cationiques constituent une vaste classe de vecteurs couramment utilisés pour la vectorisation des acides nucléiques (ADNp, siARN, ARNm) *in vitro* ou *in vivo.*

Depuis les travaux pionniers de Felgner et al. (Felgner, P.L.G.; Gadek, T.R.; Holm, M.; Roman, R.; Chan, H.W.; Wenz, M.; Northrop, J.P.; Ringold, M.G.; Danielsen, M. Proc. Natl. Acad. Sci. U.S. A., 1987, 84, 7413-7417), des efforts ont été faits pour proposer de nouvelles structures de lipides amphiphiles cationiques permettant d'améliorer l'efficacité de la transfection et d'élargir la connaissance des mécanismes de transfection.

La transfection est effectuée grâce à des agrégats supramoléculaires formés par l'association d'un lipide amphiphile cationique avec de l'ADN (lipoplexes). Après l'internalisation cellulaire de ces lipoplexes qui se produit par voie d'endocytose, la libération de la matière nucléique des endosomes vers le cytosol est nécessaire pour éviter la dégradation de la matière chargée à l'intérieur des lysosomes.

Différentes stratégies fondées sur une approche moléculaire ont été explorées pour favoriser la déstabilisation de la membrane endosomale ou agir sur la stabilité des lipoplexes après leur internalisation cellulaire.

Ainsi de nouveaux lipides amphiphiles cationiques pouvant être protonés dans les endosomes (effet d'éponge à protons) ou clivés par une réaction enzymatique ou redox dans le cytosol ont été proposés pour destabiliser la membrane endosomale.

Une autre stratégie pour améliorer l'efficacité de transfection consiste à améliorer la stabilité et les propriétés de fusion des lipoplexes (a) Ewert, K.; Slack, N.L.; Ahmad, A.; Evans, H.M.; Lin, A.J.; Samuel, C.E.; Safinya, C.R. Curr Med Chem., 2004, 11, 133-49 ; b) Dan, N.; Danino, D. Adv Colloid Interface Sci., 2014, 205, 230-9). Des travaux ont notamment rapporté une amélioration des transfections en associant des co-lipides comme la 1,2-dioleoyl-sn-glycero-3-phosphoetanolamine (DOPE) avec un lipide amphiphile cationique. Cette amélioration est attribuée à la propension de la DOPE à adopter une phase hexagonale inversée qui est connue pour être plus fusogène que les phases lamellaires.

Une autre stratégie pour produire des phases non lamellaires consiste à agir sur la forme moléculaire des lipides amphiphiles cationiques. Ewert et al. ont rapporté la synthèse des lipides amphiphiles cationiques possédant un groupe de tête dendritique (Ewert, K.K.; Evans, H.M.; Zidovska, A.; Bouxsein N.F.; Ahmad, A.; Safinya, C.R. J. Am. Chem. Soc. 2006, 128, 3998-4006). La forme de cette tête polaire cationique a induit la formation de phases hexagonales H_{I} lorsqu'ils sont inclus dans une formulation binaire. Des efficacités de transfection élevées ont été observées sur des lignées cellulaires connues pour être difficiles à transfecter. Lindberg et al ont montré que l'incorporation de deux chaînes phytanyl (chaînes C16-alkyles méthylés) dans la structure de lipo-phosphoramidate cationique produit une phase hexagonale inversée après la formulation dans l'eau (Lindberg, M.; Carmoy, N. ; Le Gall, T.; Fraix, A.; Berchel, M. ; Lorilleux, C.; Couthon-Gourvès, H. ; Bellaud, P. ; Fautrel, A. ; Jaffrès, P.A.; Lehn, P.; Montier, T. Biomaterials 2012, 33, 6240-6253). De bonnes efficacités de transfection in vivo ont été obtenues avec ce vecteur.

Malgré tous ces travaux il existe toujours un besoin pour le développement de nouveaux vecteurs.

Il est également souhaitable que ces nouveaux lipides amphiphiles soient obtenus par des voies de synthèse optimisées pour la production à grande échelle nécessaire pour des expériences in vivo. Cependant, il existe toujours un besoin pour le développement d'une nouvelle méthode de synthèse permettant d'obtenir de nouveaux lipides amphiphiles ramifiés à partir d'une modification simple des structures lipides amphiphiles existantes, permettant une forte modularité et n'impliquant pas une synthèse de novo, c'est-à-dire en synthétisant complètement la molécule souhaitée.

### DÉFINITIONS

Dans la présente invention, les termes ci-dessous sont définis de la manière suivante :
- « **agent de visualisation** » : concerne un composé ayant la capacité de visualiser une structure anatomique ou pathologique.
- « **alcényle** » : concerne toute chaîne hydrocarbonée linéaire ou ramifiée, portant au moins une double liaison, de 2 à 12 atomes de carbone, de préférence de 2 à 6 atomes de carbone, tel que par exemple éthényle, 2-propényle, 2-butényle, 3-butényle, 2-pentényle et ses isomères, 2-hexényle et ses isomères, 2,4-pentadiényl.
- « **alcynyle** » : concerne toute chaîne hydrocarbonée linéaire ou ramifiée, portant au moins une triple liaison, de 2 à 12 atomes de carbone, de préférence de 2 à 6 atomes de carbone, tel que par exemple éthynyle, 2-propynyle, 2-butynyle, 3-butynyle, 2-pentynyle et ses isomères, 2-hexynyle et ses isomères.

- « **acylamino** » : concerne les groupes -NRC(O)alkyle, -NRC(O)cycloalkyle, - NRC(O)cycloalcényl, -NRC(O)alcényl, -NRC(O)alcynyle, -NRC(O)aryle, - NRC(O)hétéroaryle and -NRC(O)hétérocyclique, dans lesquels R est un hydrogène ou un alkyle tel que défini ci-dessous.
- « **alkyle** » : concerne toute chaîne hydrocarbonée linéaire ou ramifiée saturée, de 1 à 12 atomes de carbone, de préférence de 1 à 6 atomes de carbone, tel que par exemple méthyle, éthyle, n-propyle, isopropyle, n-butyle, sec-butyle, isobutyle, tertio-butyle, pentyle et ses isomères (e.g. n-pentyle, iso-pentyle), hexyle et ses isomères (e.g. n-hexyle, iso-hexyle), et de manière encore plus préférentielle de 1 à 3 carbones, tel que par exemple méthyle, éthyle, n-propyle, isopropyle.
- « **alkyle amino** » : concerne le groupe -NHR dans lequel R est un alkyle tel que défini ci-dessus.
- « **alkylaryle** » : concerne un groupement comportant un groupement aryle tel de défini ci-dessus lié de manière covalente à un groupement alkyle tel que défini ci-dessus et relié par le groupement aryle.
- « **alkyloxy** » : concerne tout groupe -O-alkyle.
- « **alkyloxycarbonyle** » : concerne tout groupe -C(O)-O-alkyle.
- « **aminé** » : concerne le groupe -NH₂.
- « **aminocarbonyle** » : concerne les groupes -C(O)NR'R" dans lesquels R' et R" indépendamment choisi parmi le groupe comprenant hydrogène, alkyle, alcényle, alcynyle, aryle, cycloalkyle, cycloalcényle, hétéroaryle et hétérocyclique, et dans lesquels R' et R" sont éventuellement reliés entre eux avec l'azote auquel ils sont liés pour former un groupe hétérocyclique ou hétérocyclique substitué, tel que par exemple une pipérazine substituée.
- « **aminothiocarbonyle** » : concerne les groupes -C(S)NR'R" dans lesquels R' et R" indépendamment choisi parmi le groupe comprenant hydrogène, alkyle, alcényle, alcynyle, aryle, cycloalkyle, cycloalcényle, hétéroaryle et hétérocyclique, et dans lesquels R' et R" sont éventuellement reliés entre eux avec l'azote auquel ils sont liés pour former un groupe hétérocyclique ou hétérocyclique substitué, tel que par exemple une pipérazine substituée.
- « **amphiphile** » : concerne une espèce chimique possédant à la fois au moins un groupe hydrophile et au moins un groupe hydrophobe. Les triglycérides ne sont pas des espèces amphiphiles ; Selon un mode de réalisation de l'invention, les composés de formule (II) ne sont pas des triglycérides.
- « **aryle** » : concerne un système mono- ou polycyclique de 5 à 20, de préférence de 6 à 12, atomes de carbone possédant un ou plusieurs noyaux aromatiques (quand il y a deux noyaux, il est fait référence à un biaryle) parmi lesquels on peut citer le groupe phényle, le groupe biphényle, le groupe 1-naphtyle, le groupe 2-naphtyle, le groupe tétrahydronaphtyle, le groupe indanyle, et le groupe binaphtyle.
- « **arylalkyle** » : concerne un groupement comportant un groupement alkyle tel de défini ci-dessus lié de manière covalente à un groupement aryle tel que défini ci-dessus et relié par le groupement alkyle.
- « **contre-ion** » : concerne un ion mobile de signe contraire. Des exemples non-limitatifs de ces contre-ions comprennent les ions des métaux alcalins, des métaux alcalino-terreux, des métaux de transition, ammonium, pyridinium, chlorure, bromure, iodure, tosylates, triflates, méthylsulfate.
- « **cycloalkyle** » : concerne un groupement alkyle cyclique ou polycyclique, comportant de 3 à 8 atomes de carbone ; de préférence un groupement cyclopropyle, cyclopentyle ou cyclohexyle.
- « **cycloalcényle** » : concerne un groupement alcényle cyclique ou polycyclique, comportant de 3 à 8 atomes de carbone ; de préférence un groupement cyclopropèneyle, cyclopentèneyle ou cyclohexèneyle.
- « **dialkylamino** » : concerne le groupe -NRR' dans lequel R et R' sont des alkyles tel que défini ci-dessus.
- « **hétérocycle** » : concerne un groupement non aromatique, complètement saturé ou partiellement insaturé (par exemple, un composé cyclique comportant de 3 à 7 atomes, un composé bicyclique comportant de 7 à 11 atomes) qui possède au moins un hétéroatome sur l'un des cycles carbonés. Chaque cycle du groupement hétérocycle contenant un hétéroatome peut avoir 1, 2, 3 ou 4 hétéroatomes sélectionné parmi l'azote, l'oxygène et/ou le soufre, les atomes de soufre et d'azote pouvant être optionnellement oxydés et l'atome d'azote potentiellement quaternaire. Le groupe hétérocyclique peut être attaché à tout hétéroatome ou atome de carbone du cycle, où la valence le permet. Les cycles des hétérocycles polycycliques peuvent être fusionnés, pontés et / ou reliés par un ou plusieurs atomes spiro. Des exemples non-limitatifs de ces cycles comprennent les groupes aziridinyle, oxiranyle, thiiranyle, piperidinyle, azetidinyle, 2-imidazolinyle, pyrazolidinyle imidazolidinyle, isoxazolinyle, oxazolidinyle, isoxazolidinyle, thiazolidinyle, isothiazolidinyle, piperidinyle, succinimidyle, 3H-indolyle, indolinyle, isoindolinyle, 2H-pyrrolyle, 1-pyrrolinyle, 2-pyrrolinyle, 3-pyrrolinyle, pyrrolidinyle, 4H-quinolizinyle, 2-oxopiperazinyle, piperazinyle, homopiperazinyle, 2-pyrazolinyle, 3-pyrazolinyle, tetrahydro-2H- pyranyle, 2H-pyranyle, 4H-pyranyle, 3,4-dihydro-2H-pyranyle, oxetanyle, thietanyle, 3-dioxolanyle, 1 ,4-dioxanyle, 2,5-dioximidazolidinyle, 2-oxopiperidinyle, 2-oxopyrrolodinyle, indolinyle, tetrahydropyranyle, tetrahydrofuranyle, tetrahydrothiophenyle, tetrahydroquinolinyle, tetrahydroisoquinolin-1 -yle, tetrahydroisoquinolin-2-yle, tetrahydroisoquinolin-3-yle, tetrahydroisoquinolin-4-yle, thiomorpholin-4-yle, thiomorpholin-4-ylsulfoxide, thiomorpholin-4- ylsulfone, 1 , 3-dioxolanyl, 1 ,4-oxathianyle, 1 ,4-dithianyl, 1 ,3,5-trioxanyle, 1 H-pyrrolizinyle, tetrahydro-1 ,1-dioxothiophenyle, N-formylpiperazinyle, and morpholin-4-yle.
- « **hétéroaryle** » : concerne mais n'est pas limité à des cycles aromatiques de 5 à 12 atomes de carbone qui sont fusionnés ou liés de manière covalente, typiquement contenant 5 à 6 atomes; au moins un des carbones aromatiques dans au moins un des cycles est remplacé par l'oxygène, l'azote ou le soufre, l'azote et le soufre peuvent potentiellement être oxydés et l'azote potentiellement sous forme quaternaire. De tels cycles peuvent être fusionnés avec un aryle, cycloalkyle, hetéroaryle ou hétérocycle. Des exemples non-limitatifs de ces cycles comprennent les groupes pyrrolyle, furanyle, thiophenyle, pyrazolyle, imidazolyle, oxazolyle, isoxazolyle, thiazolyle, isothiazolyle, triazolyle, oxadiazolyle, thiadiazolyle, tetrazolyle, oxatriazolyle, thiatriazolyle, pyridinyle, pyrimidyle, pyrazinyle pyridazinyle, oxazinyle, dioxinyle, thiazinyle, triazinyle, imidazo[2,1-b][1 ,3]thiazolyle, thieno[3,2-b]furanyle, thieno[3,2- b]thiophenyle, thieno[2,3-d][1 ,3]thiazolyle, thieno[2,3-d]imidazolyle, tetrazolo[1,5-a]pyridinyle, indolyle, indolizinyle, isoindolyle, benzofuranyle, isobenzofuranyle, benzothiophenyle, isobenzothiophenyle, indazolyle, benzimidazolyle, 1,3-benzoxazolyle, 1,2-benzisoxazolyle, 2,1-benzisoxazolyle, 1,3-benzothiazolyle, 1 ,2-benzoisothiazolyle, 2,1-benzoisothiazolyle, benzotriazolyle, 1,2,3-benzoxadiazolyle, 2,1,3-benzoxadiazolyle, 1,2,3-benzothiadiazolyle, 2,1,3-benzothiadiazolyle, thienopyridinyle, purinyle, imidazo[1,2-a]pyridinyle, 6-oxo-pyridazin-1(6H)-yle, 2-oxopyridin-1 (2H)-yle, 6-oxo-pyrudazin-1(6H)-yle, 2-oxopyridin-1(2H)-yle, 1,3- benzodioxolyle, quinolinyle, isoquinolinyle, cinnolinyle, quinazolinyle, quinoxalinyle.
- « **hétérocycle neutre protonable** » : concerne mais n'est pas limité des cycles aromatiques de 5 à 12 atomes de carbone qui sont fusionnés ou liés de manière covalente, typiquement contenant 5 à 6 atomes; au moins un des carbones aromatiques dans au moins un des cycles est remplacé par l'azote et pouvant être protoné. Des exemples non-limitatifs de ces cycles comprennent les groupes imidazole, pyridine, pyridazine, pyrimidine, pyrazine, quinoleine, quinoxaline, indole.
- « **groupe polaire** » : concerne un groupe hydrophile.
- « **groupe réactif** » : concerne un groupe capable de réagir avec un autre groupe chimique pour former une liaison covalente, c'est à dire réactif de manière covalente sous des conditions réactionnelles appropriées, et représentant généralement un point de fixation pour une autre substance. Le groupe réactif est un groupement présent sur les composés de la présente invention qui est capable de réagir chimiquement avec un groupe fonctionnel sur un composé différent pour former une liaison covalente. Les groupes réactifs comprennent en général les groupes nucléophiles, électrophiles et les groupes photo activables.
- « **halogéno** » : concerne les groupes fluoro, chloro, bromo, ou iodo.
- « **halogène** » : concerne les atomes de fluor, chlore, brome et iode, préférentiellement concerne les atomes de chlore, de brome et d'iode.
- « **linker** » ou « **groupe de liaison** »: se réfère à une liaison covalente ou un groupement comprenant une série de liaisons covalentes stables, le groupement comportant de 1 à 40 atomes plurivalents choisis dans le groupe constitué de C, N, O, S et P ; liant de façon covalente une groupement, une fonction de couplage ou un groupe vectorisant au reste du ligand de l'invention. Le nombre d'atomes plurivalents dans un linker peut-être, par exemple, 0, 1, 2, 3, 4, 5, 6, 7, 8, 9, 10, 20, 25 ou 30. Un linker peut-être linéaire ou non-linéaire ; certains linkers ont des chaînes latérales ou des groupes fonctionnels pendants (ou les deux). Des exemples de tels chaînes latérales sont des modificateurs d'hydrophilicité, par exemple des groupes solubilisants comme, par exemple, sulfo (-S03H ou -SO3-) ou carboxylate (-COO-). Dans un mode de réalisation, un linker est composé de toute combinaison de liaisons simples, doubles, triples ou aromatiques carbone-carbone, carbone-azote, azote-azote, carbone-oxygène et carbone-soufre. Les linkers peuvent à titre d'exemple consister en une combinaison de groupements choisis parmi les groupes alkyle, - C(O)NH-, -C(O)O-, -NH-, -S-, -O-, -C(O)-, -S(O)n- où n est égal à 0, 1 ou 2 ; cycles à 5 ou 6 chaînons monocycliques et chaînes latérales fonctionnelles (par exemple sulfo, hydroxy ou carboxy). En outre, lorsque le linker lie une fonction de couplage au reste du ligand de l'invention, ladite fonction de couplage peut ensuite être mise à réagir avec une substance réactive ayant des propriétés vectorisantes, conduisant à un ligand dans lequel le linker lie un groupe vectorisant au reste du ligand. Dans ce cas, le linker contient typiquement un résidu d'une fonction de couplage (tels que par exemple le groupe carbonyle d'un ester, le groupe triazolo résultant d'une réaction click entre un azoture et un alcyne, ou le groupe -NHC(=S)NH- résultant du couplage d'une amine sur une fonction isothiocyanate). Selon un mode de réalisation, le linker se réfère à une liaison simple covalente.
- "**lipide**" : concerne une chaine carbonée linéaire ou ramifiée, saturée, insaturée ou polyinsaturée. Ce motif peut être accroché à la structure d'un amphiphile par un groupement fonctionnel.
- "**lipoplexe**" : concerne un complexe acide nucléique - liposome ; ledit acide nucléique pouvant être l'ADN, le siARN ou l'ARNm.
- **"liposome"** : concerne une vésicule artificielle formée par des bicouches lipidiques concentriques, emprisonnant entre elles des compartiments aqueux. Les liposomes sont obtenus à partir d'amphiphiles.
- par « **pharmaceutiquement acceptable** », on entend que les ingrédients d'une composition pharmaceutique sont compatibles entre eux et non délétères pour le patient.
- « **prodrogue** » : concerne des dérivés pharmacologiquement acceptables, tels que par exemple des amides ou esters, dont le produit de biotransformation in vivo génère le composé biologiquement actif. Les prodrogues sont généralement caractérisées par une augmentation de la bio-disponibilité et sont facilement métabolisées en composés biologiquement actifs in vivo.
- « **sel organique** » : concerne un composé ionique composé de cations et d'anions formant un produit neutre et sans charge nette, au moins l'un desdits ions étant de nature organique, c'est-à-dire étant un composé carboné ; Des exemples non-limitatifs de ces sels organiques comprennent les sels ammonium, phosphonium, et imidazolium.
- « **transfection** » : concerne l'introduction de matériel génétique exogène dans des cellules eucaryotes. La transfection peut être réalisée *in vitro* ou *in vivo.*
- « **UV** » : concerne la partie du spectre électromagnétique allant d'environ 300 nm à environ 400 nm.
- « **véhicule** » : concerne une substance qui porte le produit d'intérêt dans une composition, en particulier ce peut être une substance qui permet de le dissoudre. Le véhicule peut par exemple être de l'eau.
- Les linkers phosphorés sont tels que définis ci-dessous dans lequel R, R', R", R''', R'''' et R''''' sont des alkyles ou alkylene tel que défini ci-dessus :

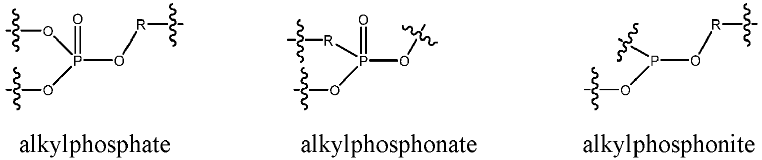

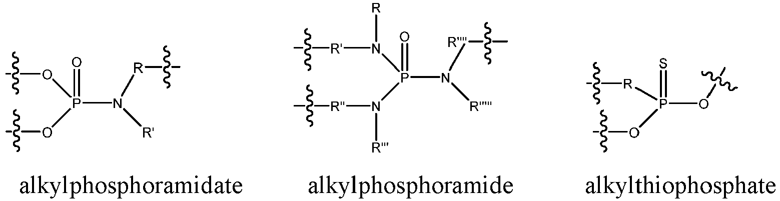

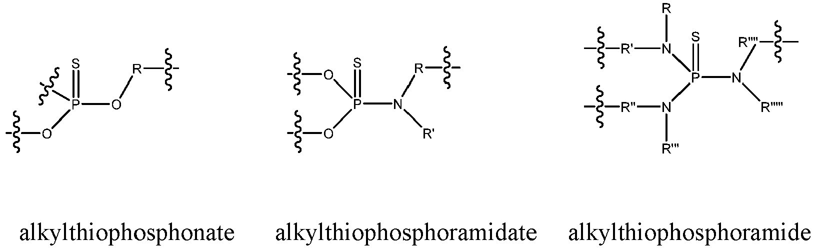

### DESCRIPTION DÉTAILLÉE

### Composés

La présente invention concerne un composé amphiphile de formule générale (II) : 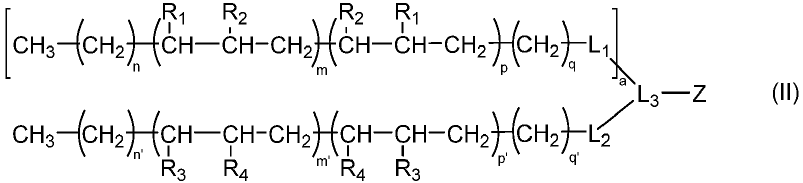 dans laquelle :
**L₁** et **L₂** représentent chacun indépendamment un linker, préférablement le linker est choisi parmi une liaison simple et les groupes alkyle, aryle, alkylaryle, arylalkyle, alkyloxy, ou alkyloxycarbonyle ;
**L₃** représente un linker, préférablement le linker est choisi parmi les groupes alkyle, alkylphosphoramidates, alkylthiophosphoramidates, alkylphosphate, alkylthiophosphate, alkylphosphonite, alkylphosphonate, alkylthiophosphonate, alkylphosphoramide, alkylthiophosphoramide, alkyloxy ou aminé ;
**Z** est un groupe fonctionnel polaire, ledit groupe étant cationique, anionique, zwitterionique ou neutre ;
**a** représente 1 ;
**n, n', q et q'** représentent chacun indépendamment un nombre entier de 1 à 15 ; **m, m', p et p'** représentent chacun indépendamment un nombre entier de 0 à 4 à condition que :
   - au moins un de m et p est différent de 0 ;
   - au moins un de m' et p' est différent de 0 ;
**R₁** et **R₂** représentent l'un un hydrogène et l'autre un groupe thioether de formule - **S-L₄-R₅** et **R₃** et **R₄** représentent l'un un hydrogène et l'autre un groupe thioether de formule **-S-L₄-R₅** tel que :
   **L₄** représente un linker, préférablement le linker est choisi parmi une liaison simple et les groupes aminocarbonyle, acylamino, alkylaminocarbonyle, aminothiocarbonyle, alkyloxycarbonyle, alkyle, aryle, cycloalkyle, alkylaryle, arylalkyle, alkyloxy, polyéthylène glycol (PEG), polypropylène glycol (PPG), un peptide ou une combinaison de ceux-ci; éventuellement interrompu ou terminé par -O-, -S-, -SO₂- ou une combinaison de ceux-ci; éventuellement comprenant en outre un résidu d'un groupe réactif par lequel **L₄** est relié à **R₅;**
   **R₅** représente un atome d'hydrogène, ou :
      - groupe polaire choisi dans le groupe des sels organiques de type ammonium, phosphonium, et imidazolium et des hétérocycles neutres protonables ;
      - un groupe réactif choisi dans le groupe comprenant le groupe N₃, amino, alkylamino, COOH, amide, maléimide, alcyne, SH, OH, ester, ester activé, acide carboxylique activé, halogéno, nitro, nitrile, isonitriles, acrylamide, aldéhyde, cétone, acétals, cétals, anhydride, thiocyanate, isothiocyanate, isocyanate, hydrazines, hydrazides, hydrazones, éthers, oxydes, cyanates, diazo, diazonium, sulfures, disulfures, sulfoxydes, sulfones, acides sulfoniques, acides sulfiniques, sulfates, acides sulféniques, amidines, imides, imines, imidates, nitrones, hydroxylamines, oximes, acides hydroxamiques, acides thiohydroxamique, allènes, ortho-esters, sulfites, énamines, ynamines, urées, pseudo-urées, semicarbazides, carbodiimides et carbamates ; ou
      - un groupe bioactif choisi parmi les amides, les esters, les amino-acides, les peptides, les protéines, les enzymes, les polysaccharides, les nucléosides, les nucléotides, les oligonucléotides, les radio-isotopes, les carboranes, et des combinaisons de ceux-ci.
sous réserve que lorsque a est égal à 0 alors Z est différent de -S-CH2-CH2-OH et de -C(O)OMe.

Le composé de l'invention présente l'avantage d'être fonctionnalisable ou fonctionnalisé. Selon un mode de réalisation, la fonctionnalisation est réalisée à partir de **R₅** lorsque **R₅** représente un groupe réactif. Selon un mode de réalisation, les hétérocycles neutres protonables représentent les groupes imidazole et pyridine.

Selon un mode de réalisation, **L₁** et **L₂** représentent chacun indépendamment une liaison simple ou un groupe alkyloxy.

Selon un mode de réalisation, **L₃** représente un linker choisi parmi les groupes alkyle, alkylphosphate, alkylphosphonate, alkylphosphoramidate, alkylthiophosphoramide, alkyloxy ou amine. Selon un mode de réalisation préféré, **L₃** représente un groupe alkylphosphoramidate. Selon un autre mode de réalisation préféré, **L₃** représente un groupe alkyloxy.

Selon un mode de réalisation, **L₄** représente un linker, préférablement le linker est choisi parmi une liaison simple et les groupes alkyles, aryle, cycloalkyle, alkylaryl, arylalkyl, polyéthylène glycol(PEG) et polypropylène glycol (PPG). Selon un mode de réalisation, **L₄** représente une liaison simple ou un linker, préférablement le linker est choisi parmi les groupes alkyle, cycloalkyle, aryl-alkyl. Dans ces modes de réalisation, **L₄** est de préférence choisi parmi les groupes C1-C12 alkyl, cycloalkyl et aryl-alkyl. De manière plus préférentielle **L₄** est choisi parmi les groupes benzyl, cyclohexyl, propyl, hexyl, heptyl, undecyl et dodecyl.

Selon un mode de réalisation, **R₅** représente un atome d'hydrogène, un groupe polaire choisi dans le groupe des sels organiques de type ammonium, phosphonium, et imidazolium et des hétérocycles neutres protonables ou un groupe réactif choisi dans le groupe OH, acide carboxylique, amine, fonction thiol protégée, azoture, aldehyde, ou un groupe bioactif choisi parmi les amides, les esters, les radio-isotopes ou les carboranes. **R₅** représente préférentiellement un atome d'hydrogène ou un groupe OH.

Selon un mode de réalisation, **Z** représente un groupe fonctionnel polaire cationique sélectionné parmi les groupes ammonium quaternaire, ammonium tertiaire, ammonium secondaire, tels que les groupes ammonium peuvent être inclus dans une chaine aliphatique, un cycle à 5 chaînons, un hétérocycle à 5 chaînons comprenant 1, 2 ou 3 atomes d'azote, un hétéroaryle à 5 chainons comprenant 1, 2, 3 ou 4 atomes d'azote, un cycle à 6 chaînons, un hétérocycle à 6 chaînons comprenant 1, 2, 3 ou 4 atomes d'azote, un hétéroaryle à 6 chainons comprenant 1 ou 2 atomes d'azote, phosphonium, tels que les groupes phosphonium peuvent être inclus dans une chaine aliphatique, un cycle à 5 chaînons, un cycle à 6 chaînons, arsonium, tels que les groupes arsonium peuvent être inclus dans une chaine aliphatique et une combinaison de ceux-ci. Selon un mode de réalisation préféré, **Z** représente un groupe fonctionnel polaire cationique sélectionné parmi les groupes ammonium quaternaire, tels que les groupes ammonium peuvent être inclus dans une chaine aliphatique, un hétéroaryle à 6 chainons comprenant 1 ou 2 atomes d'azote, phosphonium, tels que les groupes phosphonium peuvent être inclus dans une chaine aliphatique, et arsonium, tels que les groupes arsonium peuvent être inclus dans une chaine aliphatique et une combinaison de ceux-ci. Dans ces modes de réalisation, **Z** est de préférence un groupe ammonium quaternaire, phosphonium et arsonium plus préférentiellement, **Z** est un ammonium quaternaire et encore plus préférentiellement **Z** est une fonction -N⁺Me₃.

Selon un autre mode de réalisation, **Z** représente un groupe fonctionnel polaire zwitterionique sélectionné parmi les groupes aminocarboxylate, aminosulfonate, carboxybetaïne telle que le groupe ammonium peut être inclus dans une chaine aliphatique, un cycle à 5 chaînons, un hétérocycle à 5 chaînons comprenant 1, 2 ou 3 atomes d'azote, un cycle à 6 chaînons, un hétérocycle à 6 chaînons comprenant 1, 2, 3 ou 4 atomes d'azote, une fonction sulfobetaïne telle que le groupe ammonium peut être inclus dans une chaine aliphatique, un cycle à 5 chaînons, un hétérocycle à 5 chaînons comprenant 1, 2 ou 3 atomes d'azote, un cycle à 6 chaînons, un hétérocycle à 6 chaînons comprenant 1, 2, 3 ou 4 atomes d'azote, une fonction bétaïne telle que le groupe ammonium peut être inclus dans une chaine aliphatique, un cycle à 5 chaînons, un hétérocycle à 5 chaînons comprenant 1, 2 ou 3 atomes d'azote, un cycle à 6 chaînons, un hétérocycle à 6 chaînons comprenant 1, 2, 3 ou 4 atomes d'azote, une fonction phosphobetaïne telle que le groupe ammonium peut être inclus dans une chaine aliphatique , un cycle à 5 chaînons, un hétérocycle à 5 chaînons comprenant 1, 2 ou 3 atomes d'azote, un cycle à 6 chaînons, un hétérocycle à 6 chaînons comprenant 1, 2, 3 ou 4 atomes d'azote, une phosphorylcholine, une fonction phosphocholine et une combinaison de ceux-ci. Selon un mode de réalisation préféré, **Z** représente un groupe fonctionnel polaire zwitterionique sélectionné parmi une fonction sulfobetaïne telle que le groupe ammonium peut être inclus dans une chaine aliphatique, un cycle à 5 chaînons, un hétérocycle à 5 chaînons comprenant 1, 2 ou 3 atomes d'azote, un cycle à 6 chaînons, un hétérocycle à 6 chaînons comprenant 1, 2, 3 ou 4 atomes d'azote, et une fonction phosphobetaïne telle que le groupe ammonium peut être inclus dans une chaine aliphatique, un cycle à 5 chaînons, un hétérocycle à 5 chaînons comprenant 1, 2 ou 3 atomes d'azote, un cycle à 6 chaînons, un hétérocycle à 6 chaînons comprenant 1, 2, 3 ou 4 atomes d'azote. Dans ces modes de réalisation, **Z** est de préférence une fonction sulfobetaïne telle que le groupe ammonium est inclus dans une chaine aliphatique, plus préférentiellement, **Z** est une fonction -N⁺Me₂-(CH₂)₃-SO₃⁻.

Selon un autre mode de réalisation, **Z** représente un groupe fonctionnel polaire neutre sélectionné parmi les groupes amino, alkylamino, dialkylamino, tels que le groupe dialkylamino peut être inclus dans une chaine aliphatique, un cycle à 5 chaînons, un hétérocycle à 5 chaînons comprenant 1, 2 ou 3 atomes d'azote, un cycle à 6 chaînons, un hétérocycle à 6 chaînons comprenant 1, 2, 3 ou 4 atomes d'azote, polyéthylène glycol (PEG) et une combinaison de ceux-ci. Selon un mode de réalisation préféré, **Z** représente un groupe fonctionnel polaire neutre sélectionné parmi les groupes dialkylamino, tels que le groupe dialkylamino peut être inclus dans une chaine aliphatique, un cycle à 5 chaînons, un hétérocycle à 5 chaînons comprenant 1, 2 ou 3 atomes d'azote, un cycle à 6 chaînons, un hétérocycle à 6 chaînons comprenant 1, 2, 3 ou 4 atomes d'azote et polyéthylène glycol (PEG). Dans ces modes de réalisation, **Z** est de préférence un groupe dialkylamino inclus dans une chaine aliphatique ou un polyéthylène glycol, préférentiellement, **Z** est un groupe -NMe₂.

Selon un autre mode de réalisation, **Z** représente un groupe fonctionnel polaire anionique sélectionné parmi les groupes carboxylate, sulfonate, sulfate, ou phosphate et une combinaison de ceux-ci. Selon un mode de réalisation préféré, **Z** représente un groupe fonctionnel polaire anionique sélectionné parmi les groupes carboxylate, sulfate, ou phosphate.

Selon un mode de réalisation, le composé de l'invention est cationique. Selon un autre mode de réalisation, le composé est neutre. Selon un autre mode de réalisation, le composé est zwitterionique. Selon un autre mode de réalisation, le composé est anionique.

Selon un mode de réalisation, **a** est égal à 1.

Selon un mode de réalisation, **n** est égal à **n', m** égal à **m', p** égal à **p'** et **q** égal à **q'.**

Selon un mode de réalisation, la somme de **m** et **p** est égale à 2. Selon un autre mode de réalisation, la somme de **m** et **p** est égale à 1. Selon un mode de réalisation, la somme de **m'** et **p'** est égale à 2. Selon un autre mode de réalisation, la somme de **m'** et **p'** est égale à 1.

Selon un mode de réalisation, **n, n', q et q'** représentent chacun indépendamment un nombre entier de 4 à 10, préférentiellement de 6 à 9. Selon un mode de réalisation spécifique, **n** et **n'** sont égaux à 7 et **q** et **q'** sont égaux à 8.

Selon un mode de réalisation préféré, le composé de l'invention est un composé de formule (IIa) : 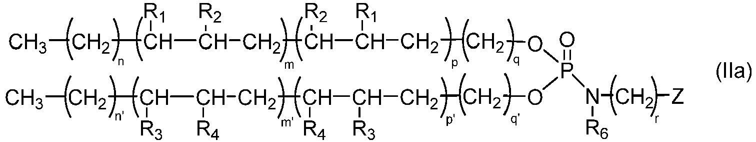 dans laquelle **R₁, R₂, R₃, R₄, Z, n, n', m, m', p, p', q** et **q'** sont tels que définis ci-dessus, **R₆** représente un hydrogène ou un alkyle et r représente un nombre entier de 1 à 10.

Selon un mode de réalisation, r représente un nombre entier de 1 à 7, préférentiellement un nombre entier de 1 à 4.

Selon un autre mode de réalisation préféré, le composé de l'invention est un composé de formule (IIb) : 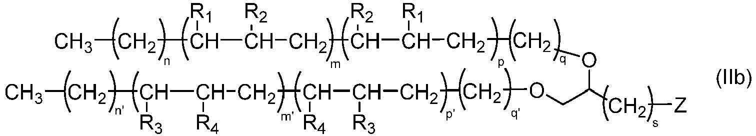 dans laquelle **R₁, R₂, R₃, R₄, Z, n, n', m, m', p, p', q** et **q'** sont tels que définis ci-dessus et s représente un nombre entier de 1 à 10.

Selon un mode de réalisation, **s** représente un nombre entier de 1 à 7, préférentiellement un nombre entier de 1 à 4.

Selon un mode de réalisation, le composé de l'invention est sélectionné dans le groupe comprenant les composés du tableau ci-dessous pour lesquels X⁻ représente un contre-ion :

| | |
|---|---|
| II-1a | 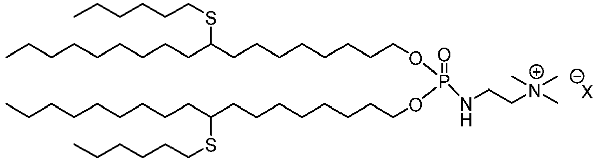 |
| II-1b | 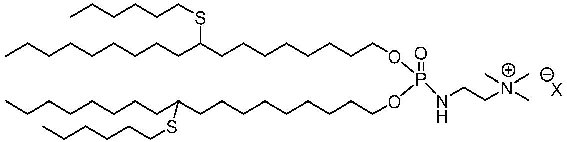 |
| II-1c | 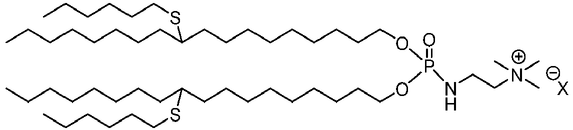 |
| II-2a | 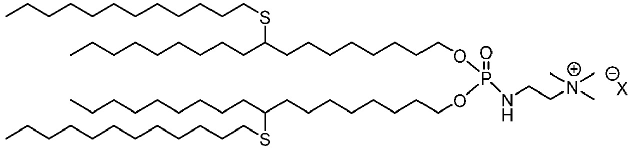 |
| II-2b | 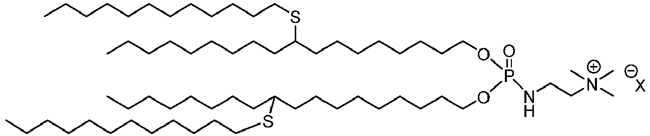 |
| II-2c | 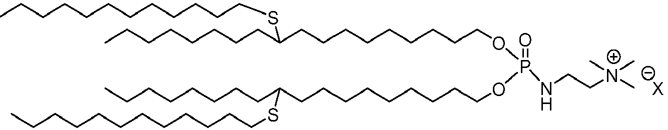 |
| II-3a | 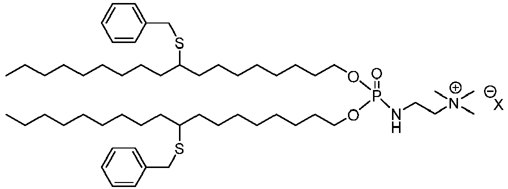 |
| II-3b | 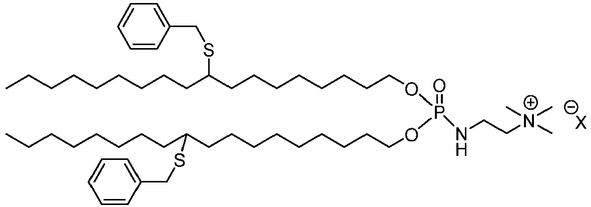 |
| II-3c | 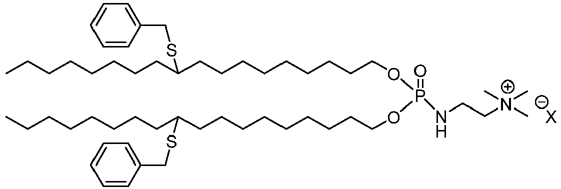 |
| II-4a | 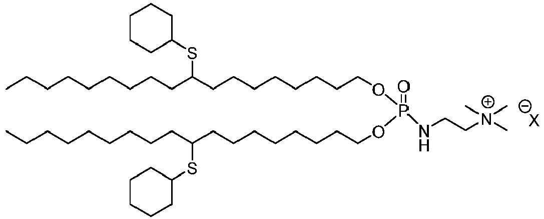 |
| II-4b | 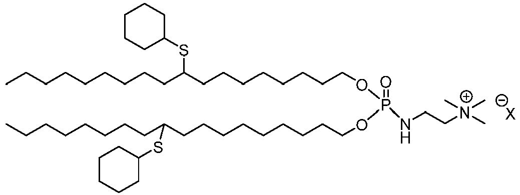 |
| II-4c | 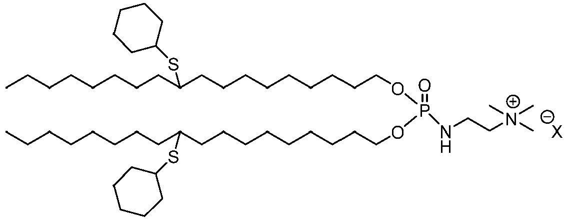 |
| II-5a | 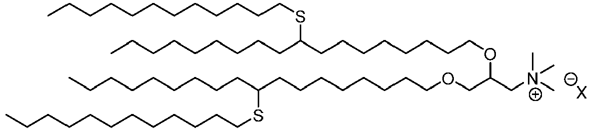 |
| II-5b | 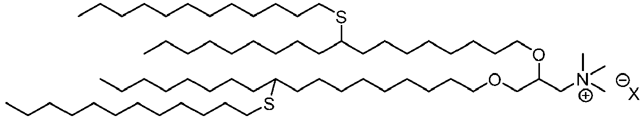 |
| II-5c | 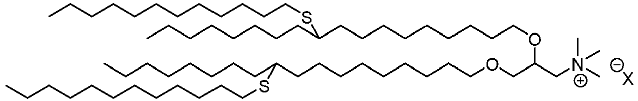 |
| II-6a | 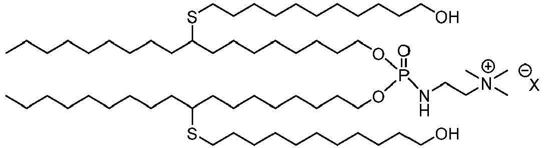 |
| II-6b | 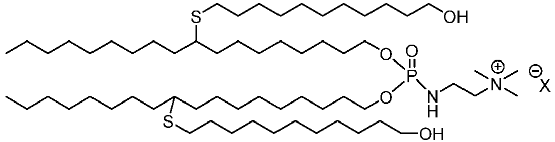 |
| II-6c | 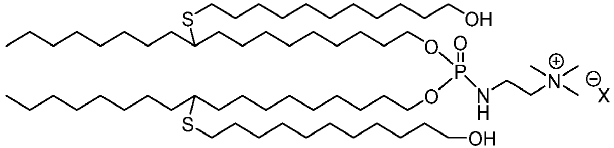 |
| II-7a | 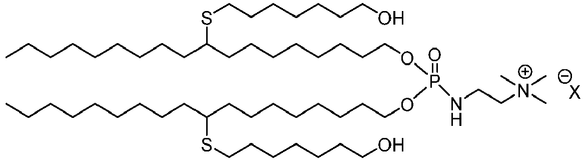 |
| II-7b | 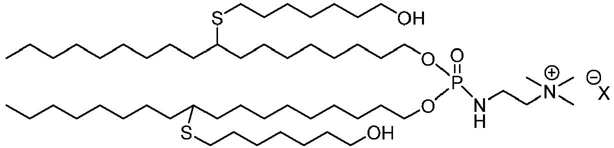 |
| II-7c | 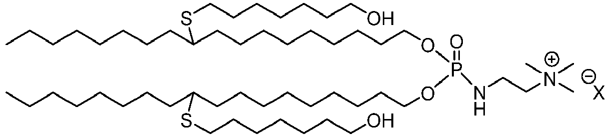 |
| II-8a | 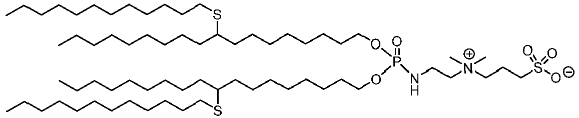 |
| II-8b | 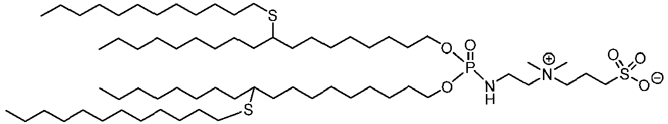 |
| II-8c | 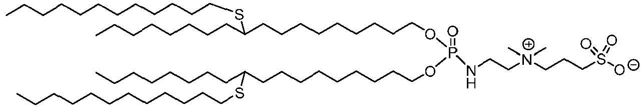 |
| II-9a | 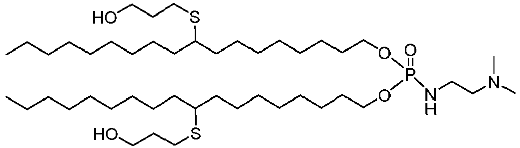 |
| II-9b | 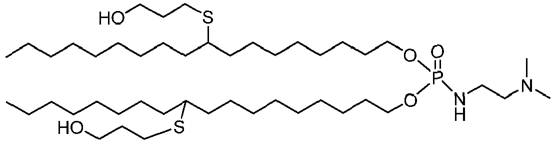 |
| II-9c | 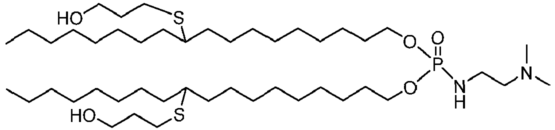 |
| II-10a | 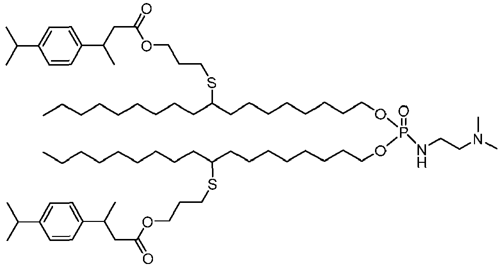 |
| II-10b | 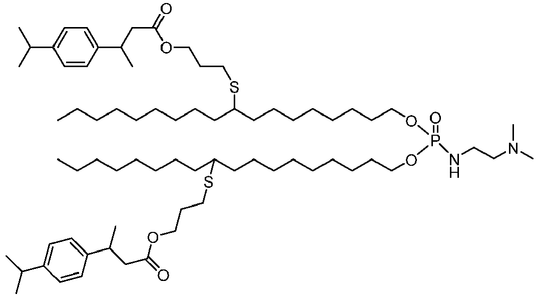 |
| II-10c | 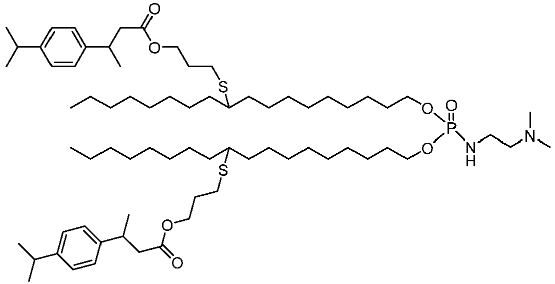 |

Dans un mode de réalisation, le contre-ion représente anion sélectionné parmi les anions chlorure, bromure, iodure, tosylates, triflates, méthylsulfate, préférentiellement le contre-ion représente un anion sélectionné parmi les anions chlorure, bromure, iodure.

Selon un mode de réalisation, le composé II-1 est un mélange de composés II-1a, II-1b et II-1c. De la même manière, le composé II-X est un mélange de composés II-Xa, II-Xb et II-Xc.

L'invention concerne également un liposome comprenant au moins un composé de l'invention tel que décrit ci-dessus. En particulier, l'invention concerne un liposome présentant une phase hexagonale et comprenant au moins l'un des composés de formule (II), (IIa) ou (IIb). Selon un mode de réalisation préféré, le liposome présente une phase hexagonale et comprend au moins un composé de formule (IIa). Selon un autre mode de réalisation préféré, le liposome présente une phase hexagonale et comprend au moins un composé de formule (IIb).

Selon un mode de réalisation, le liposome comprend uniquement des composés qui sont cationiques, c'est-à-dire avec Z étant un groupe fonctionnel polaire cationique, de formule (II), (IIa) ou (IIb). Selon un mode de réalisation préféré, le liposome comprend uniquement des composés cationiques de formule (IIa). Selon un autre mode de réalisation préféré, le liposome comprend uniquement des composés cationiques de formule (IIb).

Selon un mode de réalisation, le liposome est formé à partir d'au moins un composé neutre de formule (II), (IIa) ou (IIb) en association avec au moins un lipide amphiphile cationique non ramifié. Selon un mode de réalisation préféré, le liposome est formé à partir d'au moins un composé neutre de formule (IIa) en association avec au moins un lipide amphiphile cationique non ramifié. Selon un mode de réalisation, le lipide cationique non ramifié peut être le N-[1-(2,3- dioleyloxy)propylJ-N,N,N-trimethylammonium chloride (DOTMA) ou le composé I-1 de l'invention.

Selon un mode de réalisation, le liposome présente une phase hexagonale. Selon un mode de réalisation, le liposome présente une phase hexagonale directe. Selon un mode de réalisation, le liposome présente une phase hexagonale inverse.

Les liposomes de l'invention peuvent être obtenus selon des méthodes connues de l'homme du métier.

Selon un mode de réalisation, les liposomes peuvent être préparés par évaporation du solvant organique dans lequel sont dissous les lipides amphiphiles de l'invention, puis à les remettre en suspension dans un solvant aqueux. Cette opération doit se dérouler à une température supérieure à celle de transition de phase des lipides de l'invention.

L'invention concerne également un lipoplexe comprenant au moins un composé de l'invention tel que décrit ci-dessus. En particulier, l'invention concerne un lipoplexe présentant une phase hexagonale et comprenant au moins l'un des composés de formule (II), (IIa) ou (IIb). Selon un mode de réalisation, le lipoplexe comprend uniquement des composés qui sont cationiques, c'est-à-dire avec Z étant un groupe fonctionnel polaire cationique, de formule (II), (IIa) ou (IIb). Selon un mode de réalisation préféré, le lipoplexe comprend uniquement des composés cationiques de formule (IIa). Selon un autre mode de réalisation préféré, le lipoplexe comprend uniquement des composés cationiques de formule (IIb).

Selon un mode de réalisation, le lipoplexe est formé à partir d'au moins un composé neutre de formule (II), (IIa) ou (IIb) en association avec au moins un lipide amphiphile cationique non ramifié de formule (I). Selon un mode de réalisation préféré, le lipoplexe comprend au moins un composé neutre de formule (IIa) ainsi qu'au moins un lipide amphiphile cationique non ramifié de formule (I). Selon un mode de réalisation, le lipide cationique non ramifié peut être la DOTMA ou le composé I-1.

Selon un mode de réalisation, le lipoplexe présente une phase hexagonale. Selon un mode de réalisation, le lipoplexe présente une phase hexagonale directe. Selon un mode de réalisation, le lipoplexe présente une phase hexagonale inverse.

Les lipoplexes de l'invention peuvent être obtenus selon des méthodes connues de l'homme du métier. Les lipoplexes sont préparés en mélangeant une quantité donnée d'ADN avec les lipides amphiphiles de l'invention. Selon un mode de réalisation les rapports de charge des lipoplexes peuvent être compris entre 0.3 et 15.0, préférentiellement entre 0.5 et 8.0.

### Procédé

Les composés de l'invention peuvent être obtenus selon des réactions connues de l'homme du métier.

L'invention concerne également un procédé pour fabriquer les composés de l'invention. En particulier, l'invention concerne un procédé de synthèse chimique permettant de moduler facilement et rapidement la structure des lipides amphiphiles préexistants au niveau de leur partie lipidique au moyen d'une réaction de type thiol-ène.

L'invention concerne également le procédé pour produire un composé de formule (II), 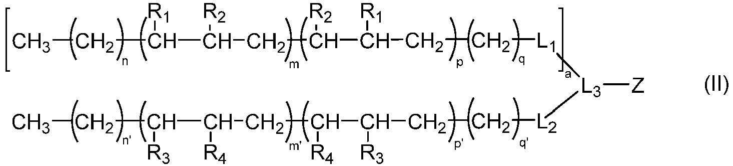 dans laquelle
**L₁** et **L₂** représentent chacun indépendamment un linker, préférablement le linker est choisi parmi une liaison simple et les groupes alkyle, aryle, alkylaryle, arylalkyle, alkyloxy, alkyloxycarbonyle ;
**L₃** représente un linker, préférablement le linker est choisi parmi les groupes alkyle, alkylphosphoramidates, alkylthiophosphoramidates, alkylphosphate, alkylthiophosphate, alkylphosphonite, alkylphosphonate, alkylthiophosphonate, alkylphosphoramide, alkylthiophosphoramide, alkyloxy ou aminé ;
**Z** est un groupe fonctionnel polaire, ledit groupe étant cationique, anionique, zwitterionique ou neutre ;
**a** représente 1 ;
**n, n', q et q'** représentent chacun indépendamment un nombre entier de 1 à 15;
**m, m', p et p'** représentent chacun indépendamment un nombre entier de 0 à 4 à condition que :
   - au moins un de m et p est différent de 0 ;
   - au moins un de m' et p' est différent de 0 ;
**R₁** et **R₂** représentent l'un un hydrogène et l'autre un groupe thioether de formule **-S-L₄-R₅** et **R₃** et **R₄** représentent l'un un hydrogène et l'autre un groupe thioether de formule **-S-L₄-R₅** dans laquelle :
   **L₄** représente un linker, préférablement le linker est choisi parmi une liaison simple et les groupes aminocarbonyle, acylamino, alkylaminocarbonyle, aminothiocarbonyle, alkyloxycarbonyle, alkyle, aryle, cycloalkyle, alkylaryle, arylalkyle, alkyloxy, polyéthylène glycol (PEG), polypropylène glycol (PPG), un peptide ou une combinaison de ceux-ci; éventuellement interrompu ou terminé par -O-, -S-, -SO₂- ou une combinaison de ceux-ci; éventuellement comprenant en outre un résidu d'un groupe réactif à travers lequel **L₄** est relié à **R₅;**
   **R₅** représente un atome d'hydrogène, ou :
      - groupe polaire choisi dans le groupe des sels organiques de type ammonium, phosphonium, et imidazolium et des hétérocycles neutres protonables ;
      - un groupe réactif choisi dans le groupe comprenant le groupe N3, amino, alkylamino, COOH, amide, maléimide, alcyne, SH, OH, ester, ester activé, acide carboxylique activé, halogéno, nitro, nitrile, isonitriles, acrylamide, aldéhyde, cétone, acétals, cétals, anhydride, thiocyanate, isothiocyanate, isocyanate, hydrazines, hydrazides, hydrazones, éthers, oxydes, cyanates, diazo, diazonium, sulfures, disulfures, sulfoxydes, sulfones, acides sulfoniques, acides sulfiniques, sulfates, acides sulféniques, amidines, imides, imines, imidates, nitrones, hydroxylamines, oximes, acides hydroxamiques, acides thiohydroxamique, allènes, ortho-esters, sulfites, énamines, ynamines, urées, pseudo-urées, semicarbazides, carbodiimides, carbamates ; ou
      - un groupe bioactif choisi parmi les amides, les esters, les amino-acides, les peptides, les protéines, les enzymes, les polysaccharides, les nucléosides, les nucléotides, les oligonucléotides, les radio-isotopes, les carboranes, et des combinaisons de ceux-ci ;
      comprenant les étapes suivantes :
      i) faire réagir un composé de formule (I) 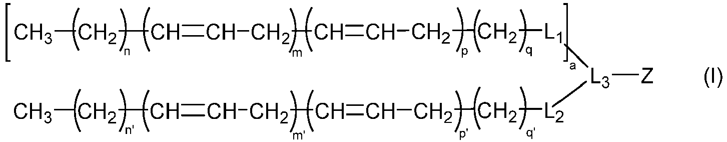
         dans laquelle **L₁, L₂, L₃, Z, a, n, n', m, m', p, p', q** et **q'** sont tels que définis ci-dessus ;
         avec un thiol **R₅-L₄**-SH dans lequel **L₄** et **R₅** tels que défini ci-dessus, en présence d'au moins un initiateur de radicaux ;
      ii) optionnellement, faire réagir le produit de la réaction (i) avec une molécule bioactive, choisie parmi les amides, les esters, les amino-acides, les peptides, les protéines, les enzymes, les polysaccharides, les nucléosides, les nucléotides, les oligonucléotides, les radio-isotopes, et les carboranes, et des combinaisons de ceux-ci.

Les modes de réalisation préférés de **L₁, L₂, L₃, L₄, Z, a, n, n', m, m', p, p', q** et **q', R₁, R₂, R₃ et R₄** définis pour le composé (II) ci-dessus s'appliquent également au procédé et aux réactifs, c'est-à-dire le composé (I) et le composé **R₅-L₄-**SH.

Selon un mode de réalisation, l'étape (i) est généralement effectuée en présence d'un excès de thiol. Selon un mode de réalisation, l'étape (i) est effectuée en présence d'une quantité de thiol comprise entre 1,1 équivalents et 50 équivalents, préférentiellement entre 1,1 et 5 équivalents, plus préférentiellement entre 1,1 et 4 équivalents. Selon un mode de réalisation, lorsque le composé de formule (I) comporte une insaturation, l'étape (i) est effectuée en présence d'une quantité de thiol supérieure ou égale à 1,1 équivalent. Selon un mode de réalisation, lorsque le composé de formule (I) comporte deux insaturations, l'étape (i) est effectuée en présence d'une quantité de thiol supérieure ou égale à 2,1 équivalents. Selon un mode de réalisation, lorsque le composé de formule (I) comporte x insaturations, l'étape (i) est effectuée en présence d'une quantité de thiol supérieure ou égale à (x +0,1) équivalents.

Selon un mode de réalisation, l'étape (i) est généralement effectuée en présence d'au moins un initiateur de radicaux. Selon un mode de réalisation, l'initiateur de radicaux peut être thermoactivable ou photoactivable, dans lequel de préférence, l'initiateur de radicaux thermoactivable est choisi dans le groupe comprenant des composés diazotés tels que l'azobisisobutyronitrile (AIBN), le 2,2'-azobis[2-(2-imidazoline-2-yl)propane]dihydrochloride, azobis (2-amidinopropane)dihydrochloride (ABAH), ou l'acide azo-bis-cyanopentanoique et l'initiateur de radicaux photo-activable est choisi dans le groupe comprenant la camphorquinone, la benzophenone, les dérivés de la benzophenone, l'acetophenone, les dérivés de l'acetophenone, tels que l'α-hydroxycycloalkyl phenyl cetones ou la 2-hydroxy-2-methyl-1-phenylpropanone, les dialcoxyacetophenones, tel que la 2,2-diméthoxy-2-phenylacetophenone, les α-hydroxy- ou a -amino-acetophenones, tel que la (4-methylthiobenzoyl)-1-methyl-1-morpholinoethane, préférentiellement l'acetophenone et les dérivés de l'acetophenone, plus préférentiellement la 2,2-diméthoxy-2-phenylacetophenone.

Selon un mode de réalisation, l'étape (i) est généralement effectuée en présence d'au moins un initiateur de radicaux thermo-activable. Selon un autre mode de réalisation, l'étape (i) est généralement effectuée en présence d'au moins un initiateur de radicaux photo-activable. Selon un mode de réalisation préférentiel, l'étape (i) est effectuée en présence de la 2,2-diméthoxy-2-phenylacetophenone. Selon un mode de réalisation préférentiel, l'étape (i) est effectuée en présence d'un initiateur de radicaux photo-activable. Selon un mode de réalisation préférentiel, l'étape (i) est effectuée en présence uniquement de la 2,2-diméthoxy-2-phenylacetophenone.

Selon un mode de réalisation, l'étape (i) est effectuée en présence de d'initiateur de radicaux à une teneur de 0.1% à 50% en mol par rapport au lipide amphiphile non ramifié de formule (I), préférentiellement, à une teneur de 0.1% à 25% en mol par rapport au lipide amphiphile non ramifié de formule (I) et de manière encore plus préférentielle à une teneur de 0.5% à 15% en mol par rapport au lipide amphiphile non ramifié de formule (I).

Selon un mode de réalisation, l'étape (i) est effectuée en l'absence de solvant. Selon un mode de réalisation, l'étape (i) est effectuée à température ambiante. Selon un mode de réalisation, l'étape (i) est effectuée sous radiation UV. Selon un mode de réalisation, l'étape (i) est effectuée sous une atmosphère inerte. Selon un mode de réalisation, le composé obtenu est purifié à l'aide de techniques chromatographiques connues de l'homme du métier.

### Utilisations

L'invention concerne une composition comprenant un composé de Formule II, un liposome et/ou un lipoplexe selon l'invention et un véhicule physiologiquement acceptable. Selon un mode de réalisation, la composition de l'invention est une composition pharmaceutique comprenant un composé de Formule II en combinaison avec un véhicule pharmaceutiquement acceptable. Selon un mode de réalisation le composé de formule II est un composé de formule IIa. Selon un autre mode de réalisation le composé de formule II est un composé de formule IIb.

L'invention concerne un médicament comprenant un composé de Formule II, un liposome et/ou un lipoplexe selon l'invention. L'invention concerne un médicament comprenant un composé de Formule II. Selon un mode de réalisation le composé de formule II est un composé de formule IIa. Selon un autre mode de réalisation le composé de formule II est un composé de formule IIb.

Les utilisations décrites ci-dessous concernent l'utilisation d'un composé de Formule II, d'une composition pharmaceutique ou d'un médicament selon la présente invention.

L'invention concerne également l'utilisation d'un composé de Formule II de l'invention ou d'un liposome de l'invention ou d'un lipoplexe de l'invention pour des applications dans lesquelles des propriétés de fusion améliorées sont recherchées.

La présente invention concerne également un liposome ou lipoplexe selon l'invention pour son utilisation pour la vectorisation *in vitro* ou *in vivo,* c'est-à-dire le transfert transmembranaire d'une molécule d'intérêt.

Selon un mode de réalisation l'invention concerne un composé de Formule II de l'invention ou du lipoplexe obtenu par formulation d'un composé de l'invention pour son utilisation en transfection *in vivo,* pour la délivrance d'amides ou d'esters, de sensibilisateurs ou d'agent de visualisation, pour la thérapie génique, des traitements topiques ou des activités bactéricides.

Selon un mode de réalisation la présente invention concerne un composé de Formule II de l'invention pour son utilisation pour la transfection de cellules *in vivo.* Selon un mode de réalisation la présente invention concerne un lipoplexe obtenu par formulation d'un composé de formule II de l'invention pour son utilisation pour la transfection de cellules *in vivo.* Selon un mode de réalisation l'invention concerne l'utilisation d'un composé de Formule II de l'invention ou du lipoplexe obtenu par formulation d'un composé de l'invention pour la transfection de cellules *in vitro.*

Selon un mode de réalisation, la présente invention concerne un composé de Formule II de l'invention pour son utilisation pour la délivrance d'amides ou d'esters, de sensibilisateurs ou d'agent de visualisation. Selon un mode de réalisation, la présente invention concerne un liposome ou lipoplexe selon l'invention pour son utilisation pour la délivrance d'amides ou d'esters, de sensibilisateurs ou d'agent de visualisation. Selon un mode de réalisation, l'invention concerne l'utilisation d'un composé de l'invention ou du lipoplexe obtenu par formulation d'un composé de l'invention pour la délivrance d'amides ou d'esters, de sensibilisateurs ou d'agent de visualisation.

Selon un mode de réalisation l'invention concerne un composé de Formule II de l'invention pour son utilisation thérapeutique en transfection, pour la délivrance d'amides ou d'esters, de sensibilisateurs ou d'agent de visualisation, pour la thérapie génique, des traitements topiques ou des activités bactéricides. Selon un mode de réalisation l'invention concerne un liposome de l'invention pour son utilisation thérapeutique en transfection, pour la délivrance d'amides ou d'esters, de sensibilisateurs ou d'agent de visualisation, pour la thérapie génique, des traitements topiques ou des activités bactéricides. Selon un mode de réalisation l'invention concerne un lipoplexe de l'invention pour son utilisation thérapeutique en transfection, pour la délivrance d'amides ou d'esters, de sensibilisateurs ou d'agent de visualisation, pour la thérapie génique, des traitements topiques ou des activités bactéricides. Selon un mode de réalisation, l'invention concerne un composé de l'invention, un liposome ou un lipoplexe obtenu par formulation d'un composé de l'invention pour son utilisation dans la thérapie génique, les traitements topiques (dermatologie, ophtalmologie) ou cosmétiques ou des activités bactéricides. Selon un mode de réalisation, l'invention concerne un composé de l'invention, un liposome ou un lipoplexe obtenu par formulation d'un composé de l'invention pour son utilisation dans la thérapie génique, les traitements topiques ou des activités bactéricides.

Selon un mode de réalisation, l'invention concerne l'utilisation d'un composé de l'invention, d'un liposome ou d'un lipoplexe obtenu par formulation d'un composé de l'invention pour des traitements cosmétiques.

Selon un mode de réalisation le composé de formule II est un composé de formule IIa. Selon un autre mode de réalisation le composé de formule II est un composé de formule IIb.

Selon un mode de réalisation, le sujet est un animal, de préférence un mammifère, plus préférentiellement un humain.

Selon un mode de réalisation, les composés de Formule II, les liposomes et lipoplexes de l'invention sont formulés dans une forme adaptée à l'injection. L'injection peut être intradermique, intramusculaire, intrapéritonéale, ou sous-cutanée Dans un mode de réalisation, les composés de Formule II, les liposomes et lipoplexes sont formulés sous la forme d'une solution, telle que par exemple une solution aqueuse stérile, une dispersion, une émulsion, une suspension. Pour prévenir toute contamination par des microorganismes, un ou plusieurs agent(s) préservateur(s) peuvent être ajoutés à la composition pharmaceutique comme par exemple des agents antibactériens et antifongiques tels que les parabènes, le chlorobutanol, le phénol, l'acide sorbique, le thiomersal et d'autres agents similaires. Il peut également être préférable d'ajouter à la composition pharmaceutique un ou plusieurs agent(s) isotonique(s) tels que des sucres ou du chlorure de sodium pour réduire la douleur provoquée par l'injection.

### BRÈVE DESCRIPTION DES FIGURES

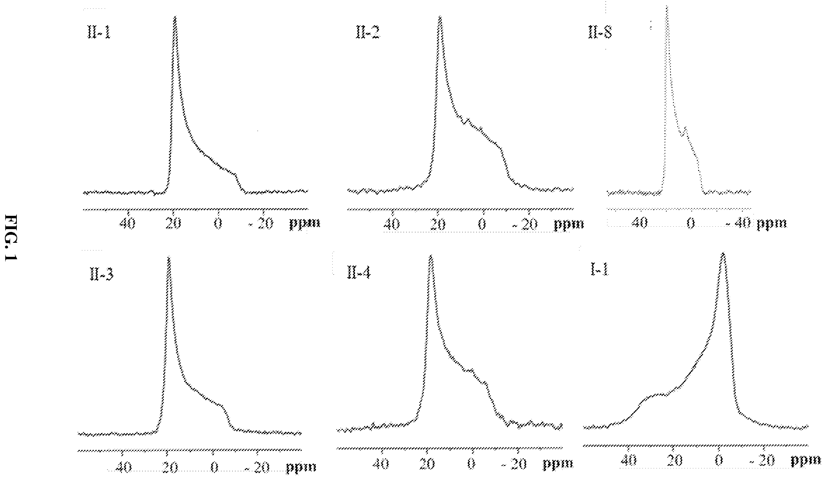

**Figure 1** est un ensemble de spectres RMN ³¹P (écho de Hahn) des composés I-1, II-1, II-2, II-3, II-4 et II-8 (en mélange avec le composé I-1 avec un ratio 1/1) réalisés à température ambiante.

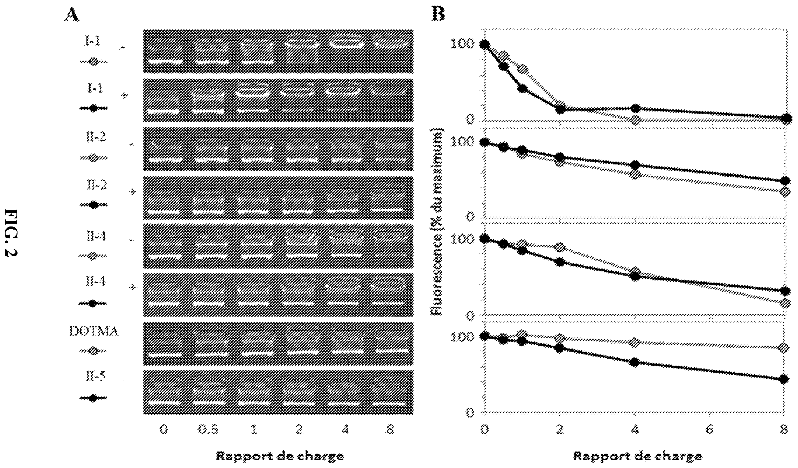

**Figure 2** concerne l'évaluation de la condensation d'ADN par les différents composés de l'invention, et formulés soit seuls (-) soit avec la DOPE (+). A représente un gel d'agarose 1% après migration (0,1 µg ADN par puits; B est un graphique représentant la fluorescence de la bande d'ADN basse par rapport au rapport de charge. La fluorescence de la bande d'ADN basse a été quantifiée puis exprimée en pourcentage de la fluorescence maximale, mesurée pour de l'ADN nu non complexé.

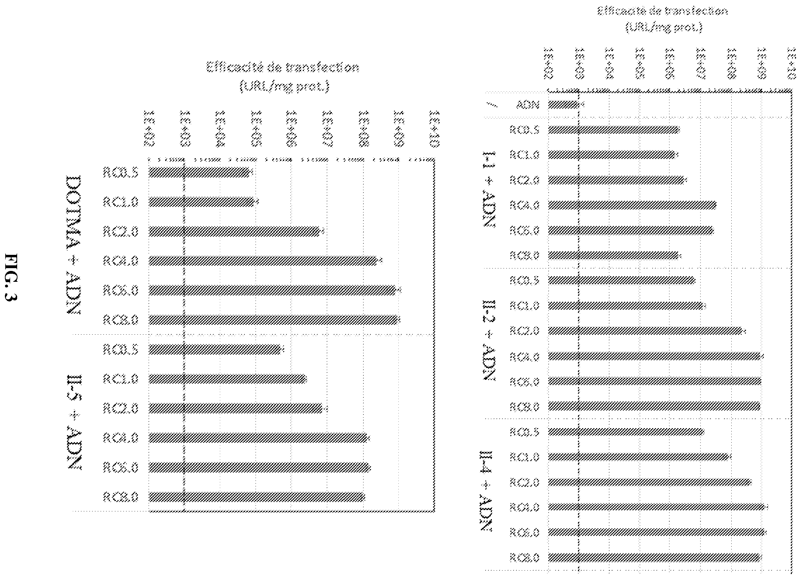

**Figure 3** est un histogramme montrant l'efficacité de transfection in vitro de cellules C2C12 au moyen des composés de l'invention et des contrôles le composé I-1 et le DOTMA. Le seuil de positivité (trait en pointillé) est déterminé avec des cellules non transfectées (exposées à de l'ADN nu, non complexé ; « ADN »). RC, rapport de charge.

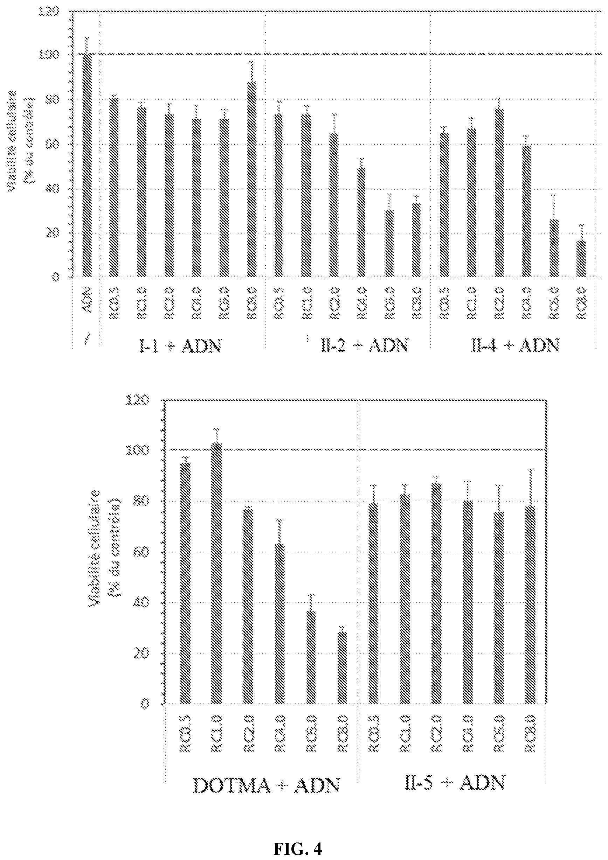

**Figure 4** est un histogramme montrant la viabilité de cellules C2C12 après transfection in vitro au moyen des composés de l'invention et des contrôles le composé I-1 et le DOTMA. La viabilité de cellules non transfectées (exposées à de l'ADN nu, non complexé ; « ADN ») est utilisée comme référence (valeur 100%, trait en pointillés). RC, rapport de charge.

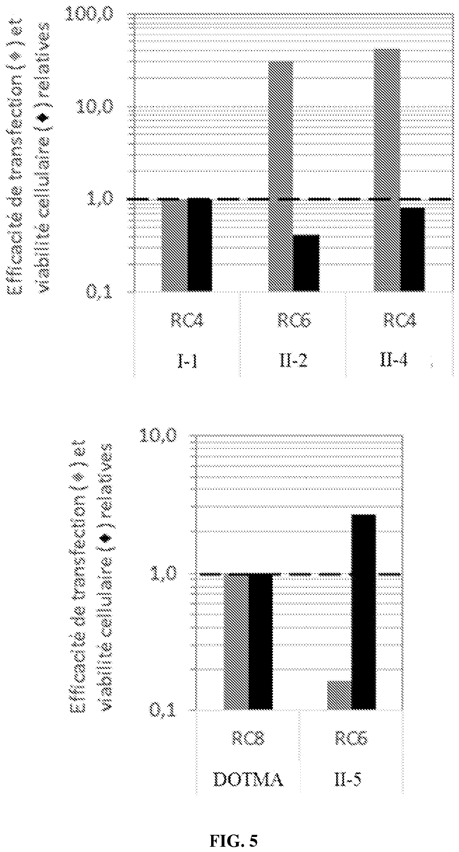

**Figure 5** est un histogramme montrant l'efficacité de transfection et viabilité cellulaire des composés à chaines grasses ramifiées exprimées par rapport à celles obtenues avec le composé à chaines grasses linéaires. Pour chaque composé, les valeurs considérées ont été celles obtenues au rapport de charge pour lequel le pic d'efficacité a été atteint.

### EXEMPLES

La présente invention se comprendra mieux à la lecture des exemples suivants qui illustrent non-limitativement l'invention.

### SYNTHÈSE

### Abréviations

ADN : acide désoxyribonucléique ;
ARN : acide ribonucléique ;
siARN : petit ARN interfèrent ;
ARNm : ARN messager ;
°C : degré Celsius ;
DOTMA : N-[1-(2,3-dioleyloxy)propylJ-N,N,N-trimethylammonium chloride;
DCC : N,N'-dicyclohexylcarbodiimide ;
DMEM : milieu minimum essentiel d'Eagle modifié par Dulbecco ;
éq. : équivalent ;
EGFP-Luc : Protéine fluorescente verte amélioré - luciférase ;
µs : microseconde ;
Ppm : parties par million ;
RC : rapport de charge ;
RMN : résonance magnétique nucléaire ;
TMS : tétraméthylsilane ;
UV : ultra-violet.

### Matériel

Les solvants utilisés ont été purifiés selon les méthodes usuelles.

Les substrats de départ ont été fournis par Aldrich, TCI ou Alfa Aesar et utilisés sans purification supplémentaire. Le 2-((bis(Z)-octadec-9-en-1-yloxy)phosphoryl)amino)-N,N,N-trimethylethanaminium iodide (réactif I-1) a été synthétisé selon la littérature. (Le Corre, S.S. ; Berchel, M. ; Belmadi, N. ; Denis, C. ; Haelters, J.P. ; Le Gall, T. ; Lehn, P. ; Montier, T. ; Jaffrès, P.A. Org. Biomol. Chem., 2014, 12, 1463-1474.).

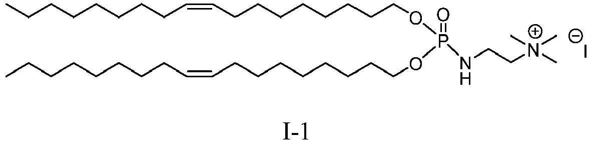

Tous les composés ont été caractérisés par spectroscopie par résonance magnétique nucléaire (RMN) ¹H (500.13 ou 400.133 ou 300.135 MHz), ¹³C (125.773 ou 75.480 MHz) and ³¹P (161.970 ou 121.498 MHz) (Spectromètres Bruker AC 300, Avance DRX 400 et Avance DRX 500). Les constantes de couplages J sont données en Hertz. Les abréviations suivantes sont utilisées : s pour singulet, d pour doublet, t pour triplet, q pour quadruplet, qt pour quintuplet, m pour multiplet, dd pour doublet de doublets et dt pour doublet de triplets.

Les composés ont également été caractérisés par spectrométrie de masse (Bruker Autoflex MALDI TOF-TOF III LRF200 CID).

### Résultats

### 1) Synthèse des réactifs zwitterioniques

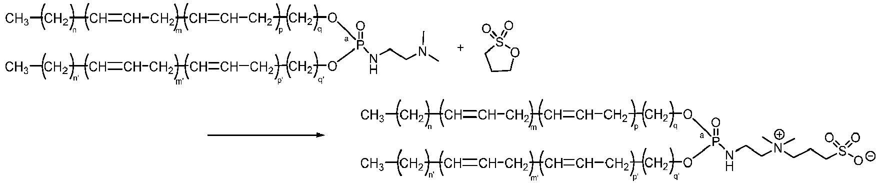

A une solution de phosphoramide de dioléyle (1 éq) dans le chloroforme, est ajoutée la propanesulfone (1,2 éq). Le milieu réactionnel est agité pendant 72 h à température ambiante. Le produit brut obtenu est ensuite purifié par chromatographie flash sur une colonne de gel de silice.

Le composé suivant a été synthétisé suivant la méthode décrite ci-dessus.

Composé 1-2 : Rendement : 44 %, **³¹P RMN :** δ (ppm, référence 85 % H₃PO₄ : 0 ppm dans CDCl₃) : 9,4 ; **¹H RMN** : δ (ppm, référence TMS : 0 ppm dans CDCl₃) : 0,85 à 0,89 (t, ³J_{H-H}= 6,8 Hz, 6 H, C**H**₃-CH₃), 1,21 à 1,29 (m, 46 H, C**H**₂ chaine grasse), 1,62 à 1,65 (m, 4 H, C**H**₂-CH₂-O-P), 1,96 à 2,01 (m, 8 H, C**H**₂-CH=CH-C**H**₂), 2,19 à 2,26 (m, 2 H, ⁺N-CH₂-C**H**₂-CH₂-S), 2,89 à 2,91 (m, 2 H, ⁺N-CH₂-CH₂-C**H**₂-S), 3,24 (s, 6 H, (C**H**₃)₂N⁺), 3,41 à 3,47 (m, 2 H, ⁺N-C**H**₂-CH₂), 3,51 à 3,61 (m, 2 H, N-CH₂-CH₂-⁺N-C**H**₂), 3,67 à 3,72 (m, 2 H, N-CH₂-C**H**₂-⁺N-CH₂), 3,90 à 3,96 (m, 4 H, C**H**₂-O-P), 4,82 à 5,00 (m, 1 H, P-N**H**), 5,29 à 5,37 (m, 4 H, C**H**=C**H**) ; **¹³C RMN :** δ (ppm, référence TMS : 0 ppm dans CDCl₃) : 14,2 (**C**H₃-CH₂), 25,5 (**C**H₂ chaine grasse), 27,2 (**C**H₂ chaine grasse), 28,9 à 30,5 (**C**H₂ chaine grasse), 31,9 (**C**H₂ chaine grasse), 51,3 ((**C**H₃)₂N⁺), 67,0 (**C**H₂-O-P), 129,9 à 130,2 (CH=CH).

### 2) Synthèse des composés de l'invention

### 2.1) Chimie click avec des lipides amphiphiles

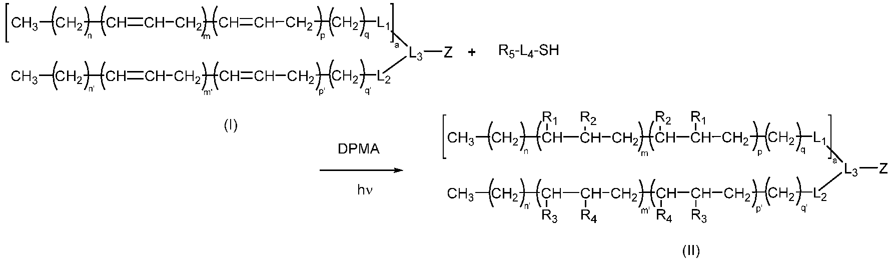

Le composé de formule (I) (1 éq) et le thiol (3,5 éq) sont mélangés dans un tube en verre, qui est placé dans un bain à ultra-sons jusqu'à la dissolution totale du composé de formule (I) (10 à 20 minutes). Le mélange est dégazé sous argon, avant l'addition de la 2,2-dimethoxy-2-phenylacetophenone (10% en masse). La solution est placée sous UV, à température ambiante, pendant 4 heures. Le produit brut obtenu est ensuite purifié par chromatographie flash sur une colonne de gel de silice.

Les composés suivants ont été synthétisés suivant la méthode décrite ci-dessus.

Composés II-1 : Rendement : 42 % (120 mg) ; **³¹P RMN :** δ (ppm, référence 85% H₃PO₄ : 0 ppm dans CHCl₃) = 8,6; **¹H RMN :** δ (ppm, référence TMS: 0 ppm dans CHCl₃) = 0,84 à 0,91 (m, 12H, C**H**₃-CH₂), 1,30 à 1,66 (m, 76H, **C**H₂ chaine grasse), 2,43 à 2,47 (t, ³J_{H-H} = 8,0Hz, 4H, C**H**₂-S), 2,50 à 2,56 (qt, ³J_{H-H} = 6Hz, 2H, (CH₂)₂C**H**-S), 3,45 (s, 9H, ⁺N(C**H**₃)₃), 3,55 à 3,63 (m, 2H, C**H**₂-NH), 3,85 à 3,88 (m, 2H, C**H**₂-⁺N(CH₃)₃), 3,95 à 3,99 (m, 4H, C**H**₂-O-P), 4,40 à 4,53 (m, 1H, N**H**); **¹³C RMN** : δ (ppm, référence TMS: 0 ppm dans CHCl₃) = 13,8 (C**H**₃-CH₂), 22,3 à 31,6 (**C**H₂ chaine grasse), 34,6 (**C**H₂-**C**H-**C**H₂), 35,9 (**C**H₂-NH), 45,6 (**C**H-S), 54,5 (⁺N(**C**H₃)₃), 66,4 (**C**H₂-⁺N(CH₃)₃, 66,8 (d, ²J_{C-P} = 5,2Hz, CH₂-O-P) ; **MALDI-TOF** : [M]⁺ calculé pour C₅₃H₁₁₂N₂O₃PS₂ = 919,785, [M]⁺ _{mesuré} = 919,775 ;

Composés II-2 : Rendement : 65 % (150 mg) ; **RMN ³¹P** : δ (ppm, référence 85% H₃PO₄ : 0 ppm dans CHCl₃) = 8,6 ; **RMN ¹H** : δ (ppm, référence TMS : 0 ppm dans CHCl₃) = 0,85 à 0,88 (t, ³J_{H-H}= 6,4 Hz, 12H, C**H**₃-CH₂), 1,24 à 1,66 (m, 76H, chaine grasse), 2,43 à 2,47 (t, ³J_{H-H} = 8,0Hz, 4H, C**H**₂-S), 2,51 à 2,55 (qt, ³J_{H-H} = 8,0Hz, 2H, (CH₂)₂C**H**-S), 3,45 (s, 9H, ⁺N(C**H**₃)₃), 3,50 à 3,61 (m, 2H, C**H**₂-NH), 3,83 à 3,86 (m, 2H, C**H**₂-⁺N(CH₃)₃), 3,95 à 4,00 (m, 4H, C**H**₂-O-P), 4,30 à 4,43 (m, 1H, N**H**) ; **RMN ¹³C** : δ (ppm, référence TMS : 0 ppm dans CHCl₃) = 13,9 (**C**H₃-CH₂), 22,5 ppm à 31,8 (**C**H₂ chaine grasse), 34,8 (**C**H₂-CH-**C**H₂), 36,0 (**C**H₂-NH), 45,8 (CH-S), 54,7 (⁺N(**C**H₃)₃), 66,6 (**C**H₂-⁺N(CH₃)₃), 66,9 (d, ²J_{C-P} = 5,3Hz, **C**H₂-O-P) ; **MALDI-TOF:** [M]⁺ _{calculé} = 1087,975 ;[M]⁺_{mesuré} pour C₆₅H₁₃₆N₂O₃PS₂ = 1087,972 ;

Composé II-3 : Rendement : 58 % (150 mg) ; **³¹P RMN** : δ(ppm, référence 85% H₃PO₄: 0 ppm dans CHCl₃) = 8,7 ; **¹H RMN** : δ (ppm, référence TMS : 0 ppm dans CHCl₃) = 0,83 à 0,91 (m, 6H, C**H**₃-CH₂), 1,25 à 1,68 (m, 60H, chaine grasse), 2,45 à 2,51 (qt, J_{H-H} = 6,4Hz, 2H, (CH₂)₂C**H**-S), 3,43 (s, 9H, ⁺N(C**H**₃)₃), 3,48 à 3,52 (m, 2H, C**H**₂-NH), 3,68 (s, 4H, C**H**₂-Ph), 3,83 à 3,86 (m, 2H, C**H**₂-⁺N(CH₃)₃), 3,96 to 4,01 (m, 4H, C**H**₂-O-P), 4,30 à 4,45 (m, 1H, N**H**), 7,19 à 7,33 (m, 10H, C**H** aromatique) ; **¹³C RMN:** δ (ppm, référence TMS : 0 ppm dans CHCl₃) = 14,1 (**C**H₃-CH₂), 22,6 à 31,8 (**C**H₂ chaine grasse), 34,5 ((**C**H₂)₂-CH-S), 35,0 (**C**H₂-Ph), 45,3 ((CH₂)₂-**C**H-S), 54,8 ((**C**H₃)₃N+), 66,6 (**C**H₂-⁺N(CH₃)₃), 67,1 (d, ²J_{P-C}= 5,3ppm, **C**H₂-O-P), 126,7 (CH aromatique), 128,3 à 129,0 (CH aromatique), 138,9 (quaternaire **C**) ; **MALDI-TOF** : [M]⁺ calculé pour C₅₅H₁₀₀N₂O₃PS₂ = 931,691 ; [M]⁺_{mesuré} = 931,686 ;

Composé II-4 : Rendement : 51 % (133 mg) ; **³¹P RMN :** δ (ppm, référence 85% H₃PO₄ : 0 ppm dans CHCl₃) = 8,6 ; **¹H RMN :** δ (ppm, référence TMS: 0 ppm dans CHCl₃) = 0,81 à 0,87 (t,³J_{H-H} = 8 Hz, 6H, C**H**₃-CH₂), 1,24 à 1,90 (m, 80H, chaine grasse), 2,58 à 2,62 (m, 4H, C**H**-S-C**H**), 3,53 (s, 9H, ⁺N(C**H**₃)₃), 3,50 à 3,60 (m, 2H, C**H**₂-NH), 3,83 à 3,85 (m, 2H, C**H**₂-⁺N(CH₃)₃), 3,93 à 3,98 (m, 4H, C**H**₂-O-P), 4,30 à 4,40 (m, 1H, N**H**) ; **¹³C RMN** : δ (ppm, référence TMS : 0 ppm dans CHCl₃) = 14,1 (C**H**₃-CH₂), 22,6 à 31,9 (**C**H₂ chaine grasse), 34,3 (**C**H₂ cyclohexyle), 35,5 ((**C**H₂)₂-CH-S), 36,2 (**C**H₂-NH), 42,6 (CH cyclohexyle), 44,2 ((CH₂)₂-CH-S), 54,9 (⁺N(**C**H₃)₃), 66,7 (**C**H₂-⁺N(CH₃)₃), 67,1 (P-O-**C**H₂) ; **MALDI-TOF** : [M]⁺ calculé pour C₅₃H₁₀₈N₂O₃PS₂ = 915,753 ; [M]⁺ mesuré = 915,741 ;

Composé II-5 : Rendement : 61 % (199 mg) ; **¹H RMN :** δ (ppm, référence TMS : 0 ppm dans CDCl₃) = 0,83 à 0,87 (t, ³J_{H-H} = 6,6 Hz, 12H), 1,23 à 1,24 (m, 73H, C**H**₂ chaine grasse), 1,35 à 1,37 (m, 12H, C**H**₂ chaine grasse), 1,46 à 1,54 (m, 16H, C**H**₂-CH₂-S, C**H**₂-CH-S and C**H**₂ chaine grasse), 2,41 à 2,45 (t, ³J_{H-H} = 7,4 Hz, 4H, C**H**₂-S), 2,48 à 2,55 (qt, ³J_{H-H} = 7,3 Hz, 2H, (CH₂)-C**H**-S), 3,37 à 3,56 (m, 15H, OC**H**₂ et ⁺N(C**H**₃)₃), 3,65 à 3,69 (m, 1H, OC**H**), 3,95 à 3,99 (m, 2H, OC**H**₂) ; **¹³C RMN:** δ (ppm, référence TMS: 0 ppm dans CDCl₃) = 14,1 (**C**H₃-CH₂), 22,7 (**C**H₂ chaine grasse), 26,0 (**C**H₂ chaine grasse), 26,2 (**C**H₂ chaine grasse), 26,8 (**C**H₂ chaine grasse), 29,0 à 29,9 (**C**H₂ chaine grasse), 30,35 (**C**H₂-S-CH), 31,90 (**C**H₂ chaine grasse), 34,91 (**C**H₂ chaine grasse), 46,88 ((CH₂)₂-CH-S), 54,79 (⁺N(**C**H₃)₃), 67,93 (O**C**H₂), 68,38 (O**C**H₂), 69,31 (O**C**H₂), 72,01 (O**C**H₂), 73,63 (OCH) ;

Composé II-6 : Rendement : 48 % (140 mg) ; **³¹P RMN :** δ (ppm, référence 85 % H₃PO₄ : 0 ppm dans CDCl₃) : 8,3 ; **¹H RMN :** δ (ppm, référence TMS : 0 ppm dans CDCl₃) : 0,85 à 0,89 (t, ³J_{H-H} = 8 Hz, 6 H, C**H**₃-CH₂), 1,24 à 1,56 (m, 75 H, C**H**₂ chaine grasse), 1,49 à 1,56 (m, 12 H, C**H**₂ chaine grasse), 1,63 à 1,66 (m, 4 H, C**H**₂-CH₂-O), 1,95 à 2,06 (m, 4 H, C**H**₂-CH₂-OH), 2,43 à 2,47 (t, ³J_{H-H}= 7,2 Hz, 4 H, C**H**₂-S), 2,51 à 2,54 (m, 2 H, C**H-**S), 3,44 (s, 9 H, (CH₃)₃N⁺), 3,48 à 3,58 (m, 2 H, ⁺N-C**H**₂-CH₂), 3,59 à 3,63 (t, ³J_{H-H}= 6,8 Hz, 4 H, C**H**₂-OH), 3,85 à 3,88 (m, 2 H, C**H**₂-O-P), 3,96 à 4,01 (m, 4 H, C**H**₂-O-P), 4,51 à 4,62 (m, 1 H, N**H**-P) ; **¹³C RMN** : δ (ppm, référence TMS: 0 ppm dans CDCl₃) : 14,1 (**C**H₃-**C**H₂), 22,6 à 36,0 (**C**H₂ chaine grasse), **C**H₂-S), 39,2 **(**CH₂-NH-P), 45,9 ((CH₂)₂-**C**H-S), 54,8 (⁺N(**C**H₃)₃), 62,8 (CH₂-**C**H₂-OH), 66,5 (**C**H₂-⁺N(CH₃)₃), 67,2 à 67,3 (d, ²J_{H-H}= 22 Hz, CH₂-O-P) ;

Composé II-7 : Rendement : 23 % (65 mg) ; **³¹P RMN:** δ (ppm, référence 85 % H₃PO₄ : 0 ppm dans CDCl₃) : 8,2; **¹H RMN :** δ (ppm, référence TMS : 0 ppm dans CDCl₃) : 0,86 à 0,89 (t, ³J_{H-H}= 6,4 Hz, 6 H, C**H**₃-CH₂), 1,24 à 1,37 (m, 68 H, C**H**₂ chaine grasse), 1,46 à 1,66 (m, 16 H, C**H**₂ chaine grasse), 2,29 à 2,40 (m, 4 H, C**H**₂-CH₂-OH), 2,44 à 2,47 (t, ³J_{H-H}= 7,2 Hz, 4 H, C**H**₂-S), 2,51 à 2,53 (m, 2 H, C**H**-S), 3,45 (s, 9 H, (C**H**₃)₃N⁺), 3,47 à 3,54 (m, 2 H, ⁺N-C**H**₂-CH₂), 3,59 à 3,63 (t, ³J_{H-H} = 6,8 Hz, 4 H, C**H**₂-OH), 3,86 à 3,88 (m, 2 H, C**H**₂-NH-P), 3,97 à 4,02 (m, 4 H, C**H**₂-O-P), 4,51 à 4,62 (m, 1 H, N**H**-P) ; **¹³C RMN :** δ (ppm, référence TMS: 0 ppm dans CDCl₃) : 14,1 (**C**H₃-CH₂), 22,7 à 36,1 (**C**H₂ chaine grasse et **C**H₂-S and CH₂-**C**H₂-OH), 45,9 (**C**H-S), 54,9 ((**C**H₃)₃N⁺), 62,8 (**C**H₂-OH), 66,5 (**C**H₂-NH-P), 67,4 à 67,5 (d, ²J_{P-C}= 21 Hz, **C**H₂-O-P) ;

Composé II-8 : Rendement : 51 %, **³¹P RMN :** δ (ppm, référence 85 % H₃PO₄ : 0 ppm dans CDCl₃) : 9,5, **¹H RMN :** δ (ppm, référence TMS : 0 ppm dans CDCl₃) : 0,86 à 0,88 (t, ³J_{H-H}= 6,8 Hz, 12 H, C**H**₃-CH₂), 1,24 à 1,57 (m, 89 H, C**H**₂ chaine grasse), 1,63 à 1,64 (m, 4 H, C**H**₂-CH₂-O-P), 2,18 à 2,26 (m, 2 H, ⁺N-CH₂-C**H**₂-CH₂-S), 2,42 à 2,46 (t, ³J_{H-H} = 7,4 Hz, 4 H,C**H**₂-S-CH(CH₂)₂), 2,49 à 2,54 (st, ³J_{H-H} = 6,2 Hz, 2 H, CH₂-S-C**H**(CH₂)₂), 2,88 à 2,90 (m, 2 H, C**H**₂-S-O), 3,22 (s, 6 H, (C**H**₃)₂N⁺), 3,42 à 3,47 (m, 2 H, C**H**₂-NH), 3,50 à 3,60 (m, 2 H, P-NH-C**H**₂), 3,66 à 3,69 (m, 2 H, ⁺N-C**H**₂-CH₂-CH₂-S), 3,91 à 3,95 (m, 4H, C**H**₂-CH₂-O-P), 4,81 à 4,91 (m, 1 H, P-N**H**) ; **¹³C RMN :** δ (ppm, référence TMS : 0 ppm dans CDCl₃) : 14,1 (**C**H₃-CH₂), 22,7 (**C**H₂ chaine grasse), 25,7 (**C**H₂ chaine grasse), 26,8 (**C**H₂ chaine grasse), 27,0 (**C**H₂ chaine grasse), 29,1 à 30,0 (**C**H₂ chaine grasse), 30,4 (**C**H₂-S-CH(CH₂)₂), 31,9 (**C**H₂ chaine grasse), 34,9 à 35,1 (**C**H₂ chaine grasse), 46,0 (CH₂-S-**C**H(CH₂)₂), 51,5 ((**C**H₃)₂N⁺), 66,9 (**C**H₂-O-P) ;

Composé II-9 : **³¹P RMN :** δ (ppm, référence 85% H₃PO₄ : 0 ppm dans CDCl₃): 10,1, **¹H RMN** : δ (ppm, référence TMS : 0 ppm dans CDCl₃): 0,85 à 0,88 (t, ³J_{H-H} = 6 Hz, 6 H, C**H**₃-CH₂), 1,27 à 1,55 (m, 56 H, C**H**₂ chaine grasse), 1,62 à 1,37 (m, 4 H, C**H**₂-CH₂-O-P), 1,79 à 1,85 (m, 4 H, C**H**₂-S-CH-(CH₂)₂), 1,99 à 2,14 (m, 2 H, HO-CH₂-C**H**₂-CH₂-S), 2,21 (s, 6 H, (C**H**₂)₂-N), 2,36 à 2,39 (t, ³J_{H-H}= 5,8 Hz, 2 H, CH₂-S-C**H**-(CH₂)₂), 2,55 à 2,61 (m, 6 H, HO-CH₂-CH₂-C**H**₂-S and CH₂-C**H**₂-N), 2,92 à 2,98 (m, 2 H, P-NH-C**H**₂), 3,20 à 3,31 (m, 1H, P-N**H**-CH₂), 3,71 à 3,76 (m, 4 H, HO-C**H**₂-CH₂-CH₂-S), 3,93 à 3,99 (m, 4 H, C**H**₂-O-P).

### 2.2) Fonctionnalisation par un groupe bioactif

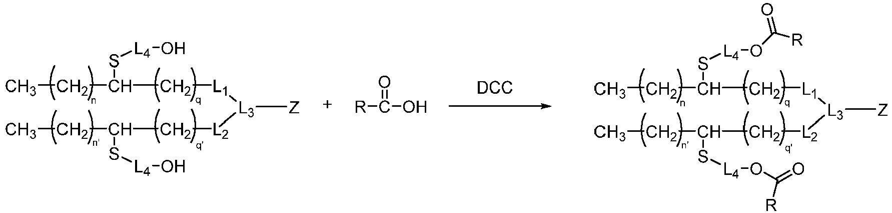

L'ester RC(O)OH (2 éq) et le composé ramifié II (1 éq) sont mélangés dans le dichlorométhane anhydre. Après l'addition du DCC (2,2 éq), le milieu réactionnel est mis sous agitation pendant une nuit. Le composé obtenu est purifié par chromatographie flash sur une colonne de gel de silice.

Le composé suivant a été synthétisé suivant la méthode C.

Composé 11-10 : Rendement : 15%, **³¹P RMN** : δ (ppm, référence 85% H₃PO₄ : 0 ppm dans CDCl₃) : 10,1 ; **¹H RMN** : δ (ppm, référence TMS : 0 ppm dans CDCl₃) : 0,85 à 0,89 (m, 18 H, C**H**₃-CH₂ and (C**H**₃)₂-CH), 1,26 à 1,48 (m, 68 H, C**H**₂ chaine grasse and C**H**₃-CH-Ph), 1,63 à 1,66 (m, 4 H, C**H**₂-CH₂-O), 1,79 à 1,84 (m, 4 H, S-CH₂-C**H**₂-CH₂-O), 2,24 (s, 6 H, (C**H**₃)₂-N), 2,38 à 2,45 (m, 12 H, C**H**₂-C(O)-O-CH₂, C**H**₂-S and C**H**-S and C**H**₂-N(CH₃)₂), 3,89 à 3,02 (m, 4 H, C**H**₂-NH-P), 3,34 à 3,42 (m, 1 H, N**H**-P), 3,66 à 3,68 (m, 2 H, CH₃-C**H**-Ph), 3,94 à 3,96 (m, 4 H, C**H**₂-O-P), 4,12 à 4,15 (t, ³J_{H-H}= 6,3 Hz, 4 H, C**H**₂-O-C(O)-CH₂), 7,06 à 7,08 (d, ³J_{H-H} = 8 Hz, 4 H, **H**² **H**⁶ / **H**^{2'} **H**^{6'}), 7,17 à 7,19 (d, ³J_{H-H}= 8 Hz, 4 H, **H**³ **H**⁵ / **H**^{3'} **H**^{5'}) ; **¹³C RMN** : δ (ppm, référence TMS : 0 ppm dans CDCl₃): 13,4 (**C**H₃-CH₂), 17,5 (**C**H₃-CH-Ph), 22,4 ((**C**H₃)₂-CH), 22,65 (**C**H₂ chaine grasse), 25,6 (**C**H₂ chaine grasse), 26,5 à 26,7 (**C**H₂ chaine grasse et **C**H₂-S), 29,0 à 31,9 (**C**H₂ chaine grasse), 34,8 (**C**H₂ chaine grasse), 38,4 (**C**H₂-NH-P), 44,8 à 46,0 (**C**H₂-N(CH₃)₂ and CH₂-N(**C**H₃)₂ and **C**H-CH₃), 59,5 (CH-S), 63,3 (**C**H₂-O-C(O)-CH₂), 66,3 (**C**H₂-O-P), 127,1 (**C**³ **C**⁵ / **C**^{3'} **C**^{5'}), 129,3 (**C**² **C**⁶ / **C**^{2'} **C**^{6'}), 137,7 (**C**⁴ / **C**^{4'}), 140,4 (**C**¹/ C^{1'}), 174,6 (CH₂-**C**(O)-O-CH₂).

### ÉTUDES DES PROPRIÉTÉS PHYSICO-CHIMIQUES DES LIPOSOMES ET LIPOPLEXES

### 1) Organisation structurale - Étude par RMN ³¹P

### - Préparation de l'échantillon

Une solution mère d'un lipide cationique est préparée dans le chloroforme. Un volume de solution mère correspondant à une masse en lipide comprise entre 50 mg et 100 mg est pipetté dans un tube à hémolyse et le chloroforme est évaporé sous flux de N₂ de façon à obtenir un film lipidique. 500 µL d'eau est ensuite ajouté et le mélange est soumis aux ultrasons jusqu'à dispersion totale du film lipidique. Le mélange est lyophilisé pendant une nuit. Un volume d'eau distillé est ensuite ajouté afin d'obtenir une solution de concentration de 100 mg.mL⁻¹. De façon à assurer l'équilibre de l'échantillon, trois cycles -198 °C / 50 °C sont effectués : l'échantillon est placé à -198 °C (azote liquide) pendant 5 min, ensuite l'échantillon est placé dans un bain marie à 50 °C pendant 30 min et passé au vortex.

### - Condition d'acquisition

Les spectres RMN ³¹P ont été acquis avec une séquence écho de Hahn (90 °-τ-180°-τ-acq). Les paramètres d'acquisition ont été les suivants : fenêtre spectrale de 200 KHz, une impulsion π/2 d'une durée de 4,88 µs, un délai de recyclage de 5 s et un délai d'écho de 40 µs. Le nombre d'acquisitions dépend du volume de l'échantillon et de la température d'acquisition, et est compris entre 128 et 1700 scans. La température de l'échantillon est équilibrée pendant 30 min avant le début de l'acquisition. Le bruit Lorentzian est filtré sur l'ensemble de la fenêtre spectrale avant la transformée de Fourier sur le sommet de l'écho. Des vésicules de dipalmitoylphosphatidylcholine (DPPC) font office de référence externe en RMN ³¹P. La déconvolution des spectres a été réalisée avec le logiciel TOPSPIN et dmfit-2009 (Massiot D, Fayon F, Capron M, King I, Le Calvé S, Alonso B, et al. Magn. Reson. Chem., 2002, 40, 70-76).

### - Résultats

La figure 1 montre très clairement par RMN ³¹P à température ambiante (Séquence écho de Hahn) que le composé non ramifié (I-1) s'auto-organise en milieu hydraté sous forme lamellaire tandis que les composés ramifiés de l'invention (ramification obtenue par réaction de type thiol-ène ; Composés II-1, II-2, II-3, II-4) génèrent des agrégats supramoléculaires adoptant une structuration hexagonale. Le spectre RMN ³¹P, enregistré dans les mêmes conditions, de la co-formulation 1/1 (ratio molaire) du composé cationique non-ramifié I-1 et du co-lipide zwitterionique et ramifié II-8, montre que le co-lipide entraine la formation d'agrégats supramoléculaires de structure hexagonale. Cette structuration peut donc être induite par la présence dans la co-formulation d'un lipide neutre (zwiterionique) ramifié par réaction thiol-ène.

### 2) Mesure de la taille et du potentiel zêta

### - Préparation des échantillons

Les lipides cationiques ont été formulés en solution liposomale par la méthode d'hydratation d'un film lipidique. Dans un premier temps, une fraction d'une solution concentrée du lipide cationique est placée dans un tube en verre et évaporée de façon à donner un film lipidique fin. 1 mL d'eau est ensuite ajouté au film et le film est hydraté pendant 3 jours à 4 °C. La solution est ensuite passée au vortex (1 min) et aux ultrasons (30 à 60 min) à 45 kHz. Ensuite 150 µL de la solution liposomale est diluée dans 1,5 mL d'eau stérile. Après filtration (200 µm) de la solution liposomale, la taille et le potentiel zêta des liposomes et lipoplexes sont déterminés.

### - Conditions d'acquisition

La taille et le potentiel zêta (ξ) des liposomes et des lipoplexes ont été mesuré avec un Zetasizer Nano ZS (Malvern Instruments) à 25 °C après une dilution appropriée des formulations. Pour les mesures des lipoplexes, chaque essai a utilisé 40 mg d'ADN plasmidique dans l'eau avec la quantité requise du lipide cationique étudié afin de former des lipoplexes avec des rapports de charge allant de 0,5 à 8,0. Pour les mesures des liposomes, une quantité de lipide équivalente à un mélange ayant un rapport de charge de 1.0 dans l'eau a été utilisée.

### - Résultats

| Composé | Taille (nm) | PdI | Potentiel Zêta (mV) | ΔPZ (mV) |
|---|---|---|---|---|
| I-1 | 94 | 0,26 | + 54 | 4 |
| II-1 | 99 | 0,26 | + 33 | 7 |
| II-2 | 148 | 0,15 | + 37 | 5 |
| II-3 | 187 | 0,36 | + 44 | 5 |
| II-4 | 137 | 0,19 | + 50 | 7 |
| II-8 + I-1 (1/1) | 136 | 0,2 | + 48 | 6 |
| II-5 | 230 | 0,30 | + 24 | 6 |
| II-6 | 188 | 0,30 | + 58 | 7 |
| II-7 | 132 | 0,20 | + 53 | 8 |

Ces données de taille montrent que les agrégats supramoléculaires formés selon la méthode d'hydratation d'un film lipidique suivie d'une étape de sonication, ont des tailles très usuelles comprises entre 90 et 230 nm. Les indices de polydispersité (PdI) témoignent du caractère relativement homogène de ces tailles dans les échantillons. Le potentiel zeta de ces échantillons, qui est toujours positif, correspond à des valeurs usuelles pour des amphiphiles cationiques ou des co-formulations comportant au moins un amphiphile cationique.

### ÉTUDE BIOLOGIQUE

### Matériel

**Cellules et conditions de culture.** La lignée cellulaire C2C12 est utilisée comme exemple ; il s'agit de myoblastes immortalisés de souris. Ces cellules sont cultivées en milieu DMEM (Lonza) supplémenté en sérum décomplémenté (10%), L-Glutamine (2 mM) et pénicilline (100 U/mL) + streptomycine (100 µg/mL). Les cellules sont maintenues dans une étuve à 37°C sous atmosphère humide et 5% de CO₂.

**ADN plasmidique.** Le plasmide rapporteur luciferase pEGFP-Luc (Clontech) est utilisé dans ces tests.

### Méthodes

**Formation des lipoplexes.** Les lipoplexes sont préparés en OptiMEM (Gibco) en mélangeant différentes quantités d'un composé donné de l'invention avec 5 µg d'ADN, afin de former des lipoplexes [lipide/ADN] caractérisés par des rapports de charges (+/-) compris entre 0.5 et 8.0.

**Condensation d'ADN.** Du bromure d'éthidium est intercalé dans l'ADN avant d'être mélangé avec les composés à tester. La condensation d'ADN est ensuite évaluée par électrophorèse en gel d'agarose. De l'ADN seul (non complexé) est utilisé comme référence.

**Transfection in vitro.** Les composés sont testés en transfection transitoire de cellules cultivées in vitro. Le protocole utilisé est celui qui a été décrit précédemment dans plusieurs publications scientifiques (Felgner P.L. et al., Proc. Natl. Acad. Sci. U.S.A. 1987, 84, 7413-7417; Le Gall T. et al., J. Med. Chem. 2010, 53, 1496-1508). Brièvement, 24 h avant la transfection proprement dite, les cellules sont ensemencées en plaques 96 puits à la densité de 12500 cellules par puits. Les complexes [lipide/ADN] sont déposés directement dans le milieu de culture des cellules, à raison de 0.25 µg d'ADN par puits. Au terme d'environ 36 h d'incubation, le milieu de culture est éliminé puis les cellules sont lysées par 75 µL de Passive Lysis Buffer (Promega) 0.5X par puits. Après 24 h de stockage à -20°C, chaque lysat est utilisé pour doser les protéines totales, mesurer l'activité luciférase et faire un test de viabilité cellulaire.

**Efficacité de transfection.** L'activité luciferase est déterminée avec le kit Luciferase Assay System (Promega). Les protéines totales sont quantifiées au moyen d'un test colorimétrique BCA assay (Interchim). L'efficacité de transfection est calculée en rapportant l'activité luciferase à la quantité de protéines totales ; elle est exprimée en unité relative de lumière par mg de protéines (URL/mg).

**Viabilité cellulaire.** La viabilité cellulaire est évaluée au moyen du kit Vialight (Lonza). Des cellules témoins (non traitées) sont utilisées comme référence (viabilité 100%).

### Résultats

La capacité des composés à condenser un ADN est évaluée en utilisant les propriétés de fluorescence de ce dernier lorsqu'il est incubé en présence de bromure d'éthidium. La condensation d'ADN s'accompagne d'une exclusion du bromure d'éthidium intercalé. La réduction de fluorescence qui en résulte permet ainsi de renseigner sur le degré de compaction de l'ADN obtenu grâce à un composé donné.

La Figure 2 montre que les composés ramifiés de l'invention compactent l'ADN mais de façon moins complète que les composés non ramifiés (I-1).

Les lipoplexes sont ensuite utilisés en transfection de cellules in vitro. La Figure 3 illustre les résultats obtenus en transfection in vitro de cellules C2C12 au moyen de lipoplexes formés à partir de différents lipides amphiphiles cationiques ramifiés selon l'invention (II-2, II-4 et II-5) ou de lipides amphiphiles cationiques non ramifiés tel que le I-1 ou le DOTMA.

Tous les composés testés se révèlent efficaces pour délivrer de l'ADN plasmidique à l'intérieur de cellules C2C12, des cellules connues pour être relativement difficiles à transfecter. Cependant, comparativement au composé à chaines grasses linéaires I-1, les composés à chaines grasses ramifiées II-2 et II-4 permettent d'obtenir des efficacités de transfection significativement plus élevées, avec un gain pouvant atteindre jusque 40 fois la référence (Figure 3). En outre, ces meilleures efficacités sont obtenues tout en conservant une bonne viabilité cellulaire comme le montre la Figure 4.

Comparativement au DOTMA, le dérivé à chaines grasses ramifiées II-5 atteint des efficacités de transfections moins élevées mais il permet de maintenir une bonne viabilité cellulaire, même à rapport de charge élevé (Figure 5).

Ces résultats indiquent qu'une ramification des chaines grasses des lipides cationiques peut permettre d'augmenter le pouvoir transfectant et/ou d'améliorer la biocompatibilité, c'est-à-dire réduire sa toxicité, selon le lipide cationique de départ et la ramification introduite.

## Revendications

1. Composé amphiphile de formule générale (II) : 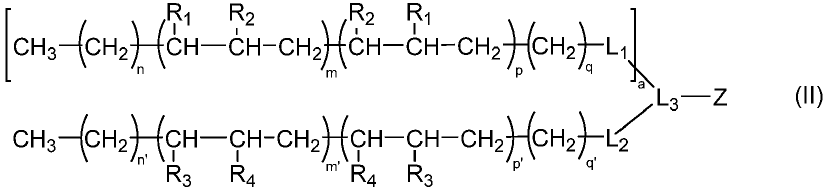 dans laquelle :
**L₁** et **L₂** représentent chacun indépendamment un linker, préférablement le linker est choisi parmi une liaison simple et les groupes alkyle, aryle, alkylaryle, arylalkyle, alkyloxy, alkyloxycarbonyle ;
**L₃** représente un linker, préférablement le linker est choisi parmi les groupes alkyle, alkylphosphoramidates, alkylthiophosphoramidates, alkylphosphate, alkylthiophosphate, alkylphosphonite, alkylphosphonate, alkylthiophosphonate, alkylphosphoramide, alkylthiophosphoramide, alkyloxy ou aminé ;
**Z** est un groupe fonctionnel polaire, ledit groupe étant cationique, anionique, zwitterionique ou neutre ;
**a** représente 1 ;
**n, n', q et q'** représentent chacun indépendamment un nombre entier de 1 à 15 ;
**m, m', p et p'** représentent chacun indépendamment un nombre entier de 0 à 4 à condition que :
- au moins un de m et p est différent de 0 ;
- au moins un de m' et p' est différent de 0 ;
**R₁** et **R₂** représentent l'un un hydrogène et l'autre un groupe thioether de formule - **S-L₄-R₅** et **R₃** et **R₄** représentent l'un un hydrogène et l'autre un groupe thioether de formule **-S-L₄-R₅** tel que :
**L₄** représente un linker, préférablement le linker est choisi parmi une laison simple et les groupes aminocarbonyle, acylamino, alkylaminocarbonyle, aminothiocarbonyle, alkyloxycarbonyle, alkyle, aryle, cycloalkyle, alkylaryle, arylalkyle, alkyloxy, polyéthylène glycol (PEG), polypropylène glycol (PPG), un peptide ou une combinaison de ceux-ci; éventuellement interrompu ou terminé par -O-, -S-, -SO₂- ou une combinaison de ceux-ci; éventuellement comprenant en outre un résidu d'un groupe réactif pars lequel **L₄** est relié à **R₅;**
**R₅** représente un atome d'hydrogène, ou :
- groupe polaire choisi dans le groupe des sels organiques de type ammonium, phosphonium, et imidazolium et des hétérocycles neutres protonables ;
- un groupe réactif choisi dans le groupe comprenant le groupe N₃, amino, alkylamino, COOH, amide, maléimide, alcyne, SH, OH, ester, ester activé, acide carboxylique activé, halogéno, nitro, nitrile, isonitriles, acrylamide, aldéhyde, cétone, acétals, cétals, anhydride, thiocyanate, isothiocyanate, isocyanate, hydrazines, hydrazides, hydrazones, éthers, oxydes, cyanates, diazo, diazonium, sulfures, disulfures, sulfoxydes, sulfones, acides sulfoniques, acides sulfiniques, sulfates, acides sulféniques, amidines, imides, imines, imidates, nitrones, hydroxylamines, oximes, acides hydroxamiques, acides thiohydroxamique, allènes, ortho-esters, sulfites, énamines, ynamines, urées, pseudo-urées, semicarbazides, carbodiimides et carbamates ; ou
- un groupe bioactif choisi parmi les amides, les esters, les amino-acides, les peptides, les protéines, les enzymes, les polysaccharides, les nucléosides, les nucléotides, les oligonucléotides, les radio-isotopes, les carboranes, et des combinaisons de ceux-ci.
sous réserve que lorsque a est égal à 0 alors Z est différent de -S-CH2-CH2-OH et de -C(O)OMe.

2. Composé selon la revendication 1, de formule (IIa) : 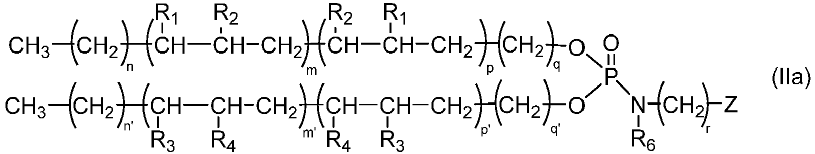
dans laquelle **R₁, R₂, R₃, R₄, Z, n, n', m, m', p, p', q** et **q'** sont tels que définis dans la revendication **1** ;
**R₆** représente un hydrogène ou un alkyle ;
**r** représente un nombre entier de 1 à 10.

3. Composé selon la revendication 1, de formule (IIb) : 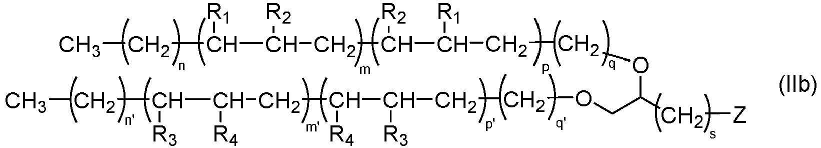
dans laquelle **R₁, R₂, R₃, R₄, Z, n, n', m, m', p, p', q** et **q'** sont tels que définis dans la revendication **1** ;
s représente un nombre entier de 1 à 10.

4. Composé selon l'une quelconque des revendications **1** à **3,** sélectionné dans le groupe comprenant les composés :
| | |
|---|---|
| II-1a | 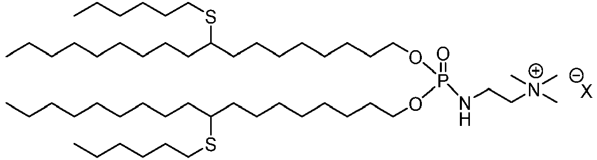 |
| II-1b | 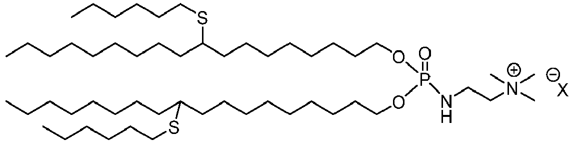 |
| II-1c | 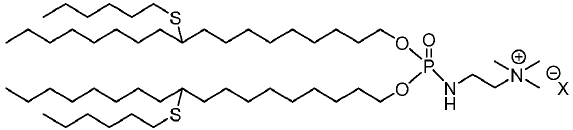 |
| II-2a | 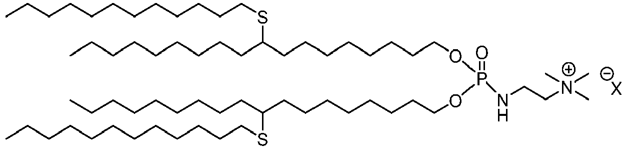 |
| II-2b | 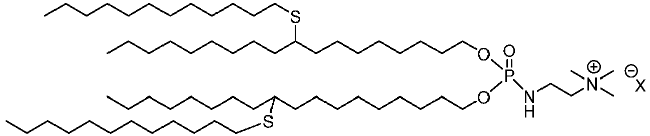 |
| II-2c | 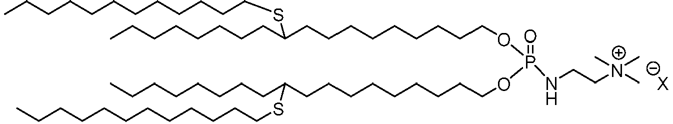 |
| II-3a | 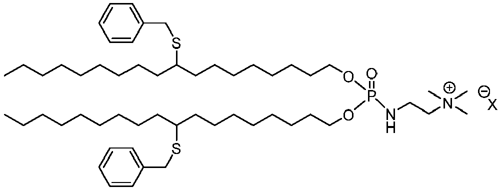 |
| II-3b | 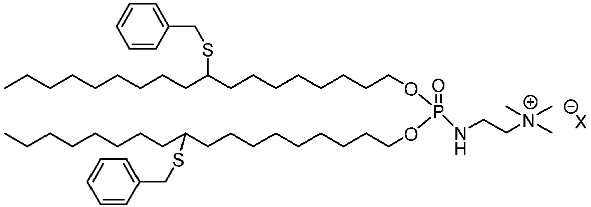 |
| II-3c | 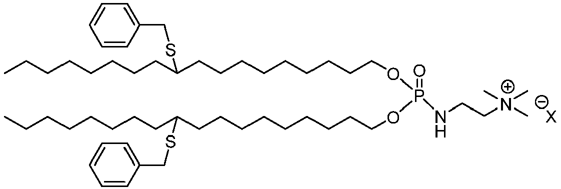 |
| II-4a | 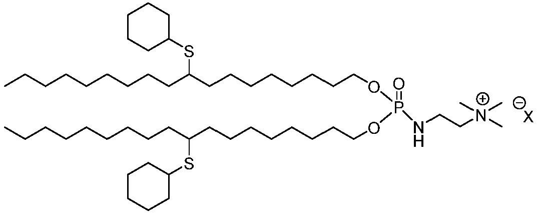 |
| II-4b | 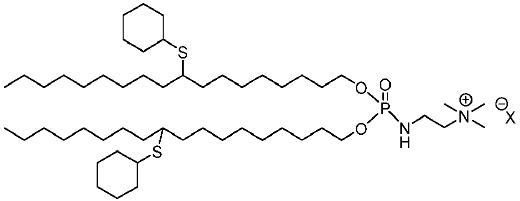 |
| II-4c | 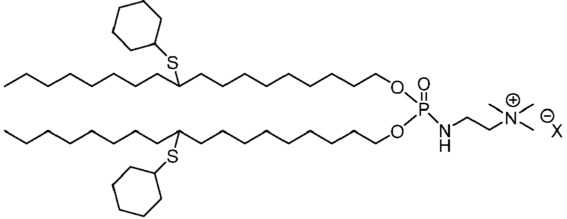 |
| II-5a | 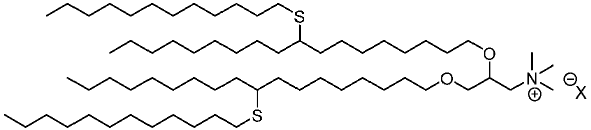 |
| II-5b | 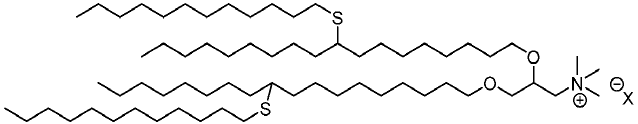 |
| II-5c | 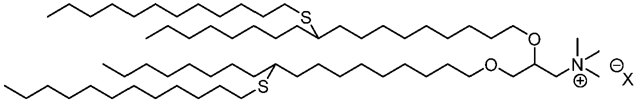 |
| II-6a | 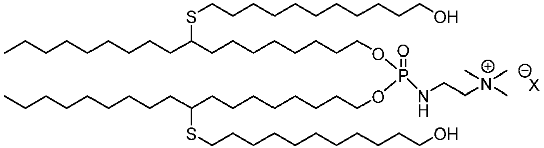 |
| II-6b | 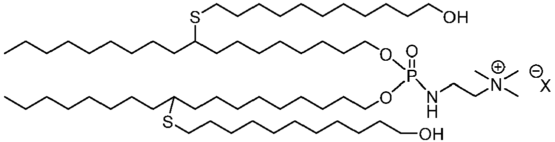 |
| II-6c | 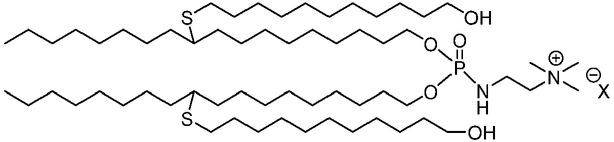 |
| II-7a | 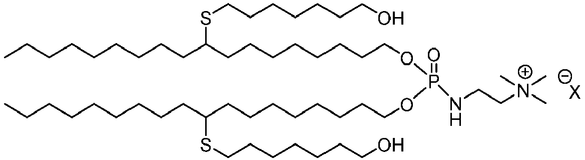 |
| II-7b | 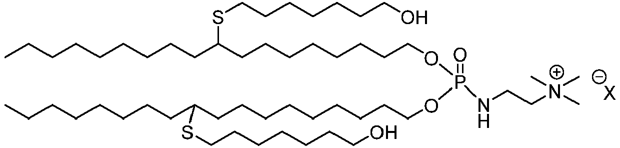 |
| II-7c | 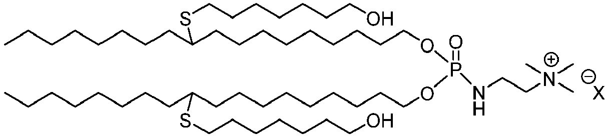 |
| II-8a | 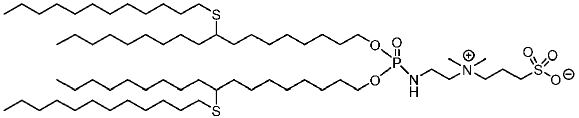 |
| II-8b | 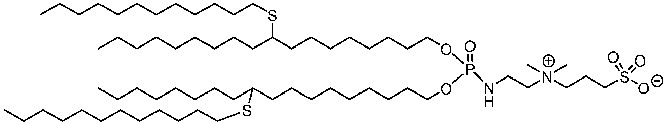 |
| II-8c | 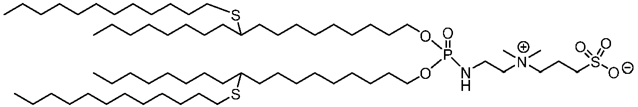 |
| II-9a | 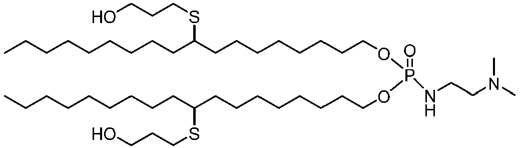 |
| II-9b | 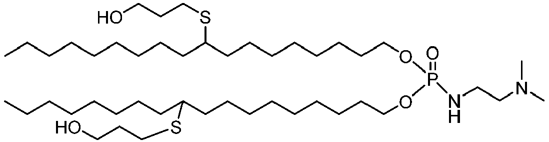 |
| II-9c | 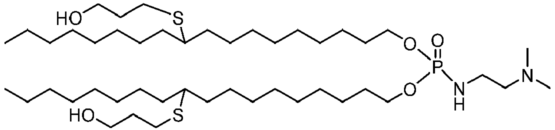 |
| II-10a | 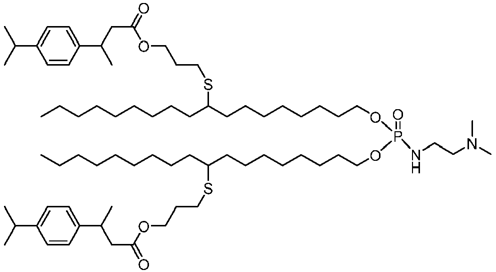 |
| II-10b | 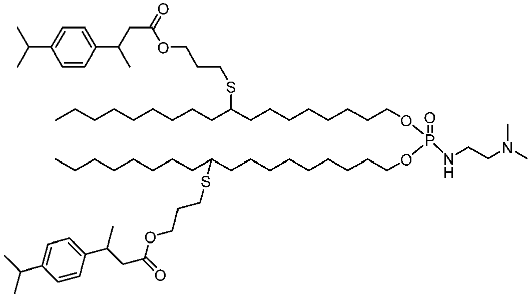 |
| II-10c | 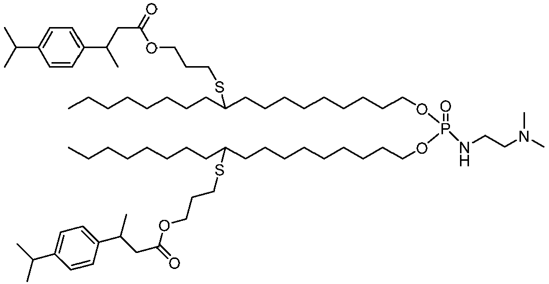 |
dans lequel X⁻ représente un contre-ion.

5. Liposome comprenant au moins l'un des composés selon l'une quelconque des revendications **1** à **4 caractérisé en ce qu'**il présente une phase hexagonale.

6. Lipoplexe comprenant au moins l'un des composés selon l'une quelconque des revendications **1** à **4 caractérisé en ce qu'**il présente une phase hexagonale.

7. Procédé pour produire un composé de formule (II) : 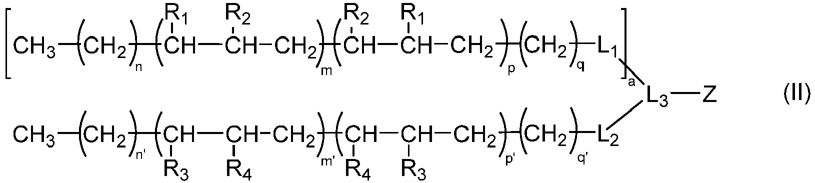 dans laquelle :
**L₁** et **L₂** représentent chacun indépendamment un linker, préférablement le linker est choisi parmi une liaison simple et les groupes alkyle, aryle, alkylaryle, arylalkyle, alkyloxy, alkyloxycarbonyle ;
**L₃** représente un linker, préférablement le linker est choisi parmi les groupes alkyle, alkylphosphoramidates, alkylthiophosphoramidates, alkylphosphate, alkylthiophosphate, alkylphosphonite, alkylphosphonate, alkylthiophosphonate, alkylphosphoramide, alkylthiophosphoramide, alkyloxy ou aminé ;
**Z** est un groupe fonctionnel polaire, ledit groupe étant cationique, anionique, zwitterionique ou neutre ;
**a** représente 1 ;
**n, n', q et q'** représentent chacun indépendamment un nombre entier de 1 à 15 ;
**m, m', p et p'** représentent chacun indépendamment un nombre entier de 0 à 4 à condition que :
- au moins un de m et p est différent de 0 ;
- au moins un de m' et p' est différent de 0 ;
**R₁** et **R₂** représentent l'un un hydrogène et l'autre un groupe thioether de formule - **S-L₄-R₅** et **R₃** et **R₄** représentent l'un un hydrogène et l'autre un groupe thioether de formule **-S-L₄-R₅** dans laquelle :
**L₄** représente un linker, préférablement le linker est choisi parmi une liaison simple et les groupes aminocarbonyle, acylamino, alkylaminocarbonyle, aminothiocarbonyle, alkyloxycarbonyle, alkyle, aryle, cycloalkyle, alkylaryle, arylalkyle, alkyloxy, polyéthylène glycol (PEG), polypropylène glycol (PPG), un peptide ou une combinaison de ceux-ci; éventuellement interrompu ou terminé par -O-, -S-, -SO₂- ou une combinaison de ceux-ci; éventuellement comprenant en outre un résidu d'un groupe réactif à travers lequel **L₄** est relié à **R₅**;
**R₅** représente un atome d'hydrogène, ou :
- groupe polaire choisi dans le groupe des sels organiques de type ammonium, phosphonium, et imidazolium et des hétérocycles neutres protonables ;
- un groupe réactif choisi dans le groupe comprenant le groupe N3, amino, alkylamino, COOH, amide, maléimide, alcyne, SH, OH, ester, ester activé, acide carboxylique activé, halogéno, nitro, nitrile, isonitriles, acrylamide, aldéhyde, cétone, acétals, cétals, anhydride, thiocyanate, isothiocyanate, isocyanate, hydrazines, hydrazides, hydrazones, éthers, oxydes, cyanates, diazo, diazonium, sulfures, disulfures, sulfoxydes, sulfones, acides sulfoniques, acides sulfiniques, sulfates, acides sulféniques, amidines, imides, imines, imidates, nitrones, hydroxylamines, oximes, acides hydroxamiques, acides thiohydroxamique, allènes, ortho-esters, sulfites, énamines, ynamines, urées, pseudo-urées, semicarbazides, carbodiimides, carbamates ; ou
- un groupe bioactif choisi parmi les amides, les esters, les amino-acides, les peptides, les protéines, les enzymes, les polysaccharides, les nucléosides, les nucléotides, les oligonucléotides, les radio-isotopes, les carboranes, et des combinaisons de ceux-ci ;
comprenant les étapes suivantes :
i) faire réagir un composé de formule (I) : 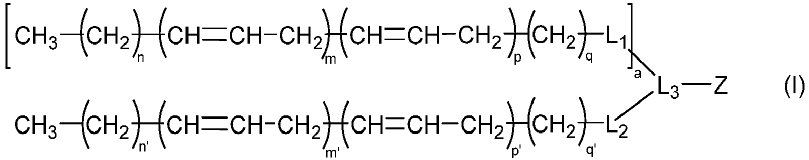 dans laquelle a, **L₁, L₂, L₃, Z, n, n', m, m', p, p', q** et **q'** sont tels que définis ci-dessus ;
avec un composé **R₅-L₄**-SH dans lequel **L₄** et **R₅** sont tels que défini ci-dessus, en présence d'au moins un initiateur de radicaux avec ;
ii) optionnellement, faire réagir le produit de la réaction (i) avec une molécule bioactive, choisie parmi les amides, les esters, les amino-acides, les peptides, les protéines, les enzymes, les polysaccharides, les nucléosides, les nucléotides, les oligonucléotides, les radio-isotopes, et les carboranes, et des combinaisons de ceux-ci.

8. Composition pharmaceutique comprenant un composé selon l'une quelconque des revendications **1** à **4** ou d'un liposome selon la revendication **5** ou d'un lipoplexe selon la revendication **6** et un véhicule physiologiquement acceptable.

9. Médicament comprenant un composé selon l'une quelconque des revendications **1** à **4** ou d'un liposome selon la revendication **5** ou d'un lipoplexe selon la revendication **6.**

10. Utilisation d'un composé selon l'une quelconque des revendications **1** à **4** ou d'un liposome selon la revendication **5** ou d'un lipoplexe selon la revendication **6** pour la vectorisation in vitro.

11. Utilisation d'un composé selon l'une quelconque des revendications **1** à **4** ou d'un liposome selon la revendication **5** ou d'un lipoplexe selon la revendication **6** pour la transfection in vitro.

12. Composé selon l'une quelconque des revendications **1** à **4** pour son utilisation en vectorisation in vivo.

13. Lipoplexe selon la revendication **6** pour son utilisation en vectorisation in vivo.

14. Composé selon l'une quelconque des revendications **1** à **4** pour son utilisation en transfection in vivo, pour la délivrance de prodrogues, de sensibilisateurs ou d'agent de visualisation, pour la thérapie génique, des traitements topiques ou des activités bactéricides.

15. Liposome selon la revendication **5** pour son utilisation en transfection, pour la délivrance de prodrogues, de sensibilisateurs ou d'agent de visualisation, pour la thérapie génique, des traitements topiques ou des activités bactéricides.

16. Lipoplexe selon la revendication **6** pour son utilisation en transfection, pour la délivrance de prodrogues, de sensibilisateurs ou d'agent de visualisation, pour la thérapie génique, des traitements topiques ou des activités bactéricides.

## Patentansprüche

1. Amphiphile Verbindung der allgemeinen Formel (II): 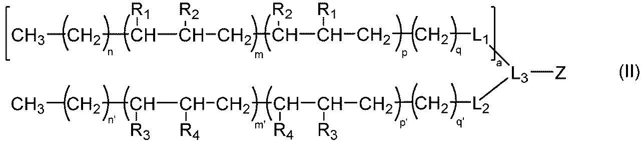 bei der:
**L₁** und **L₂** jeweils unabhängig voneinander einen Linker repräsentieren, wobei der Linker vorzugsweise aus einer einfachen Verbindung und den Alkyl-, Aryl-, Alkylaryl-, Arylalkyl-, Alkyloxy-, Alkyloxycarbonyl-Gruppen ausgewählt wird;
**L₃** einen Linker repräsentiert, wobei der Linker vorzugsweise aus den Alkyl-, Alkylphosphoramidat-, Alkylthiophosphoramidat-, Alkylphosphat-, Alkylthiophosphat-, Alkylphosphonit-, Alkylphosphonat-, Alkylthiophosphonat-, Alkylphosphoramid-, Alkylthiophosphoramid-, Alkoxy- oder Amin-Gruppen ausgewählt wird;
**Z** eine polare funktionelle Gruppe ist, wobei die Gruppe kationisch, anionisch, zwitterionisch oder neutral ist;
**a** 1 repräsentiert;
**n, n', q und q'** jeweils unabhängig voneinander eine ganze Zahl von 1 bis 15 repräsentieren;
**m, m', p und p'** jeweils unabhängig voneinander eine ganze Zahl von 0 bis 4 repräsentieren, unter der Bedingung, dass:
- mindestens eines von m und p ungleich 0 ist;
- mindestens eines von m' und p' ungleich 0 ist;
**R₁** und **R₂** einer einen Wasserstoff und der andere eine Tioether-Gruppe der Formel **-S-L₄-R₅** repräsentieren und **R₃** und **R₄** der eine Wasserstoff und der andere eine Tioether-Gruppe der Formel **-S-L₄-R₅** repräsentieren, sodass:
**L₄** einen Linker repräsentiert, wobei der Linker vorzugsweise aus einer einfachen Verbindung und den Aminocarbonyl-, Acylamino-, Alkylaminocarbonyl-, Aminothiocarbonyl-, Alkoxycarbonyl-, Alkyl-, Aryl-, Cycloalkyl-, Alkylaryl-, Arylalkyl-, Alkyloxy-, Polyethylenglykol-(PRG-), Polypropylenglykol-(PPG-) Gruppen, einem Peptid oder einer Kombination derselben ausgewählt wird; eventuell unterbrochen oder abgeschlossen durch -O-, -S-, -SO₂- oder einer Kombination derselben; eventuell weiter einen Rest einer reaktiven Gruppe, durch die **L₄** mit **R₅** verbunden ist;
**R₅** ein Wasserstoffatom repräsentiert, oder:
- eine polare Gruppe, ausgewählt aus der Gruppe der organischen Salze vom Typ Ammonium, Phosphonium und Imidazolium und protonierbare neutrale Heterozyklen;
- eine reaktive Gruppe, ausgewählt aus der Gruppe, umfassend die N₃-, Amino-, Alkylamino-, COOH-, Amid-, Maleimid-, Alcyn-, SH-, OH-, Ester-, aktivierten Ester-, aktivierter Carboxylsäure-, Halogen-, Nitro-, Nitril-, Isonitril-, Acrylamid-, Aldehyd-, Ceton-, Acetal-, Cetal-, Anhydrid-, Thiocyanat-, Isothiocyanat-, Isocyanat-, Hydrazin-, Hydrazid-, Hydrazon-, Ether-, Oxyd-, Cyanat-, Diazo-, Diazonium-, Sulfid-, Disulfid-, Sulfoxyd-, Sulfon-, Sulfonsäure-, Sulfinsäure-, Sulfat-, Sulfensäure-, Aminid-, Imid-, Imin-, Imidat-, Nitron-, Hydroxylamin-, Oxim-, Hydroxaminsäure-, Thiohydroxyamidsäure-, Allen-, Ortho-Ester-, Sulfit-, Enamin-, Ynamin-, Harnstoff-, Pseudo-Harnstoff-, Semicarbazid-, Carbodiimid- und Carbamat-Gruppe; oder
- eine bioaktive Gruppe, ausgewählt aus Amiden, Estern, Aminosäuren, Peptiden, Proteinen, Enzymen, Polysacchariden, Nukleosiden, Nukleotiden, Oligonukleotiden, Radio-Isotopen, Carboranen, und Kombinationen derselben, unter dem Vorbehalt, dass, wenn a gleich 0 ist, Z unterschiedlich von -S-CH₂-CH₂-OH und von -C(O)OMe ist.

2. Verbindung nach Anspruch **1,** der Formel (IIa): 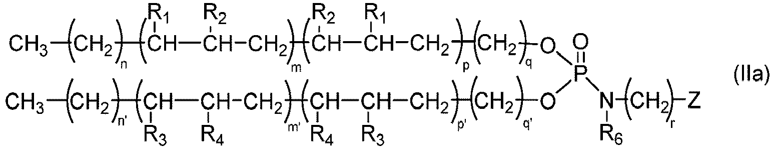
bei der **R₁, R₂, R₃, R₄, Z, n, n', m, m', p, p', q** und **q'** wie in Anspruch 1 definiert sind;
**R₆** Wasserstoff oder ein Alkyl repräsentiert;
**r** eine ganze Zahl von 1 bis 10 repräsentiert.

3. Verbindung nach Anspruch **1,** der Formel (IIb): 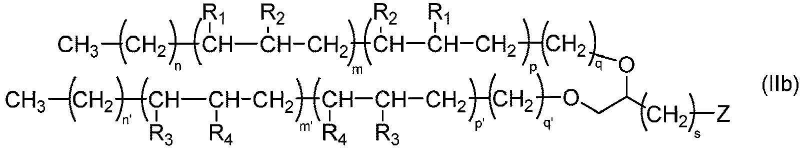
bei der **R₁, R₂, R₃, R₄, Z, n, n', m, m', p, p', q** und **q'** wie in Anspruch **1** definiert sind;
s eine ganze Zahl von 1 bis 10 repräsentiert.

4. Verbindung nach einem der Ansprüche **1** bis **3,** ausgewählt aus der Gruppe, die folgenden Verbindungen umfassend:
| | |
|---|---|
| II-1a | 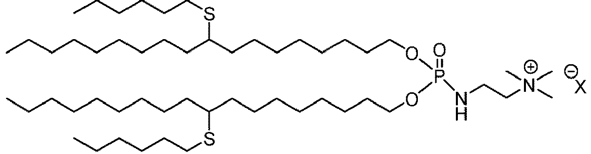 |
| II-1b | 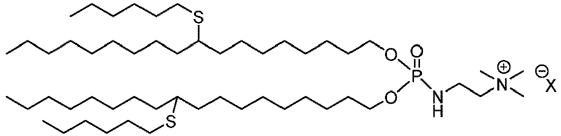 |
| II-1c | 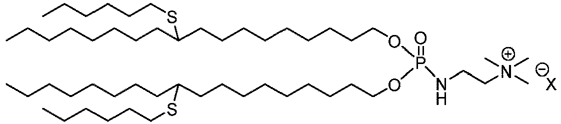 |
| II-2a | 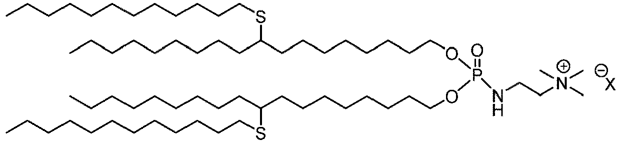 |
| II-2b | 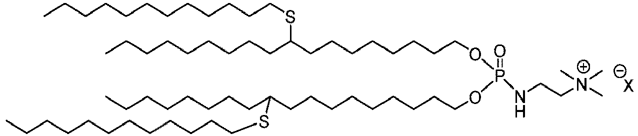 |
| II-2c | 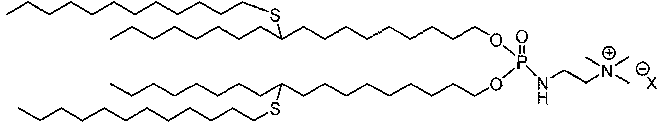 |
| II-3a | 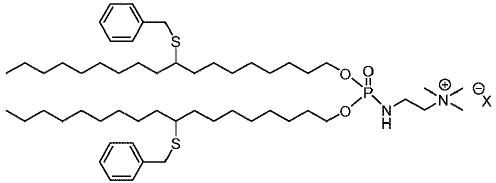 |
| II-3b | 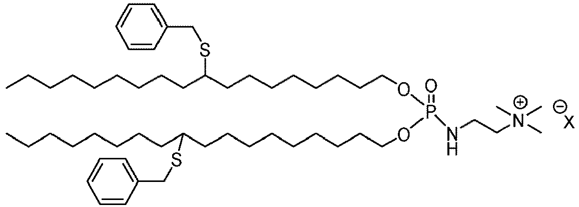 |
| II-3c | 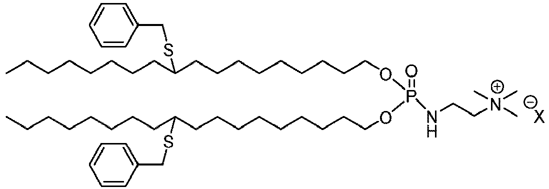 |
| II-4a | 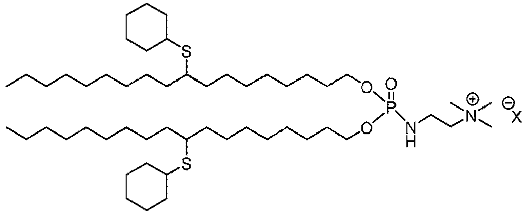 |
| II-4b | 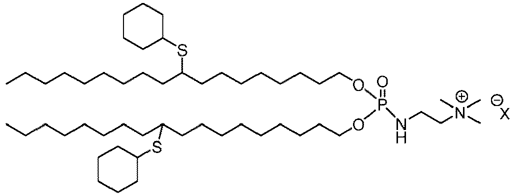 |
| II-4c | 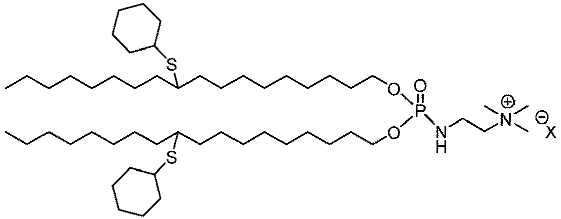 |
| II-5a | 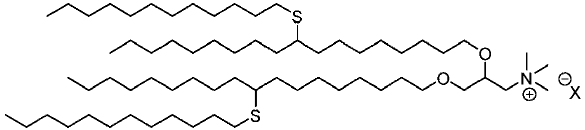 |
| II-5b | 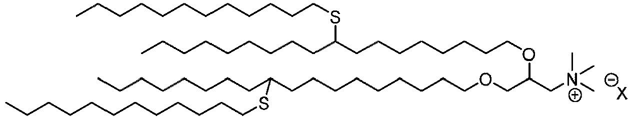 |
| II-5c | 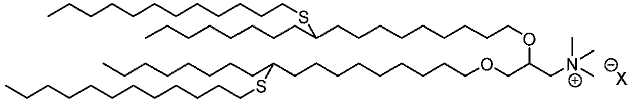 |
| II-6a | 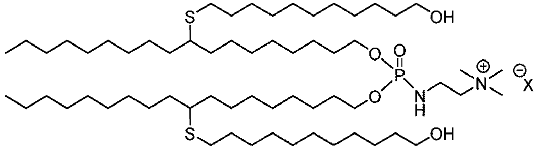 |
| II-6b | 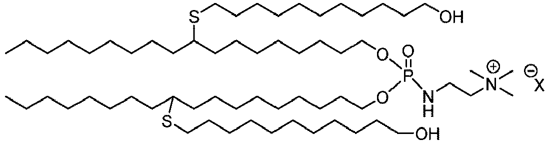 |
| II-6c | 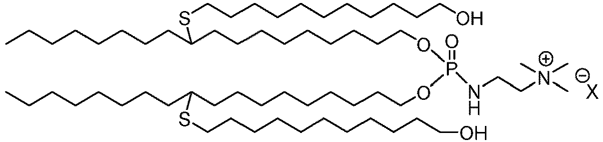 |
| II-7a | 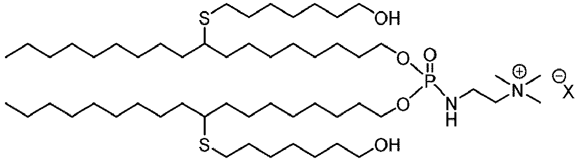 |
| II-7b | 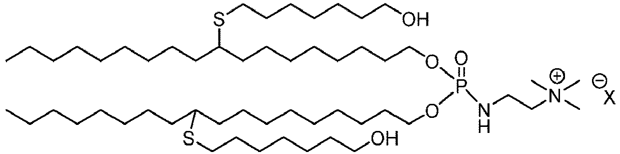 |
| II-7c | 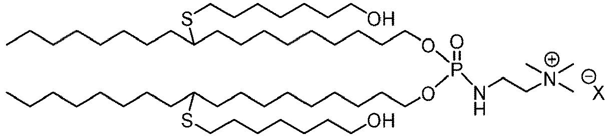 |
| II-8a | 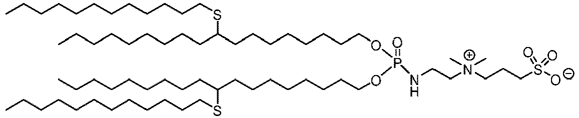 |
| II-8b | 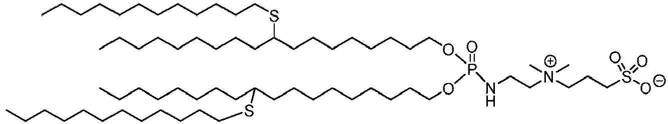 |
| II-8c | 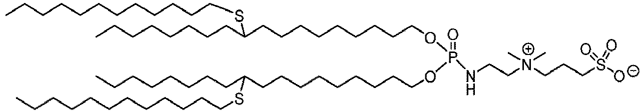 |
| II-9a | 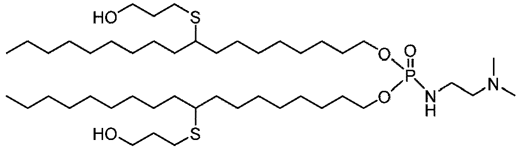 |
| II-9b | 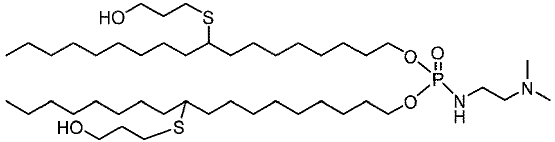 |
| II-9c | 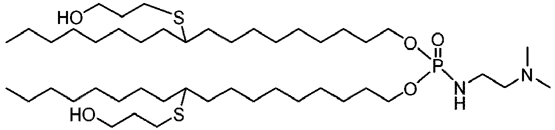 |
| II-10a | 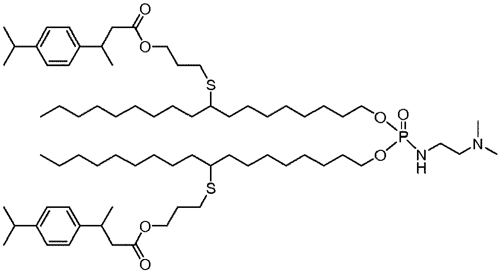 |
| II-10b | 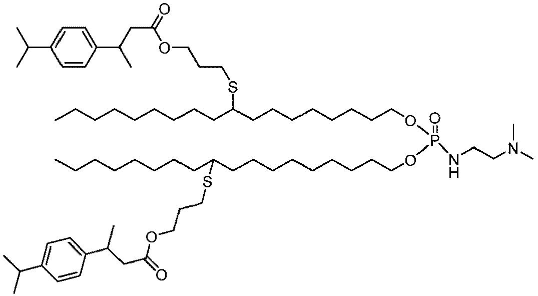 |
| II-10c | 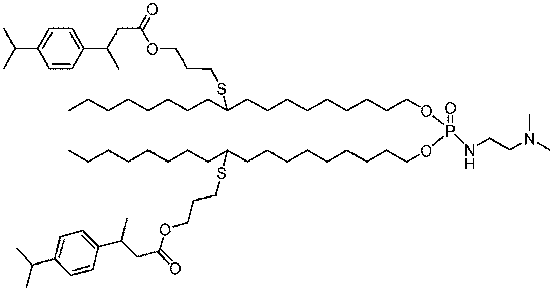 |
bei denen X⁻ ein Gegenion repräsentiert.

5. Liposom, mindestens eine der Verbindungen nach einem der Ansprüche **1** bis **4** umfassend, **dadurch gekennzeichnet, dass** es eine hexagonale Phase aufweist.

6. Lipoplex, mindestens eine der Verbindungen nach einem der Ansprüche **1** bis **4** umfassend, **dadurch gekennzeichnet, dass** es eine hexagonale Phase aufweist.

7. Verfahren zur Herstellung einer Verbindung der Formel (II): 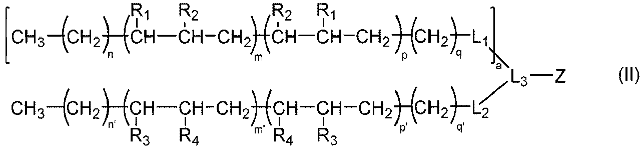 bei der:
**L₁** und **L₂** jeweils unabhängig voneinander einen Linker repräsentieren, wobei der Linker vorzugsweise aus einer einfachen Verbindung und den Alkyl-, Aryl-, Alkylaryl-, Arylalkyl-, Alkyloxy-, Alkyloxycarbonyl-Gruppe ausgewählt wird;
**L₃** einen Linker repräsentiert, wobei der Linker vorzugsweise aus den Alkyl-, Alkylphosphoramidaten-, Alkylthiophosphoramidaten-, Alkylphosphat-, Alkylthiophosphat-, Alkylphosphonit-, Alkylphosphonat-, Alkylthiophosphonat-, Alkylphosphoramid-, Alkylthiophosphoramid-, Alkoxy- oder Amin-Gruppen ausgewählt wird;
**Z** eine polare funktionelle Gruppe ist, wobei die Gruppe kationisch, anionisch, zwitterionisch oder neutral ist;
**a** 1 repräsentiert;
**n, n', q und q'** jeweils unabhängig voneinander eine ganze Zahl von 1 bis 15 repräsentieren;
**m, m', p und p'** jeweils unabhängig voneinander eine ganze Zahl von 0 bis 4 repräsentieren, unter der Bedingung, dass:
- mindestens eines von m und p ungleich 0 ist;
- mindestens eines von m' und p' ungleich 0 ist;
**R₁** und **R₂** einer einen Wasserstoff und der andere eine Tioether-Gruppe der Formel **-S-L₄-R₅** repräsentieren und **R₃** und **R₄** der eine Wasserstoff und der andere eine Tioether-Gruppe der Formel **-S-L₄-R₅** repräsentieren, sodass:
**L₄** einen Linker repräsentiert, wobei der Linker vorzugsweise aus einer einfachen Verbindung und den Aminocarbonyl-, Acylamino-, Alkylaminocarbonyl-, Aminothiocarbonyl-, Alkoxycarbonyl-, Alkyl-, Aryl-, Cycloalkyl-, Alkylaryl-, Arylalkyl-, Alkyloxy-, Polyethylenglykol-(PRG-), Polypropylenglykol-(PPG-) Gruppen, einem Peptid oder einer Kombination derselben ausgewählt wird; eventuell unterbrochen oder abgeschlossen durch -O-, -S-, -SO₂- oder einer Kombination derselben; eventuell weiter einen Rest einer reaktiven Gruppe, durch die **L₄** mit **R₅** verbunden ist;
**R₅** ein Wasserstoffatom repräsentiert, oder:
- eine polare Gruppe, ausgewählt aus der Gruppe der organischen Salze vom Typ Ammonium, Phosphonium und Imidazolium und protonierbare neutrale Heterozyklen;
- eine reaktive Gruppe, ausgewählt aus der Gruppe, umfassend die N₃-, Amino-, Alkylamino-, COOH-, Amid-, Maleimid-, Alcyn-, SH-, OH-, Ester-, aktivierten Ester-, aktivierter Carboxylsäure-, Halogen-, Nitro-, Nitril-, Isonitril-, Acrylamid-, Aldehyd-, Ceton-, Acetal-, Cetal-, Anhydrid-, Thiocyanat-, Isothiocyanat-, Isocyanat-, Hydrazin-, Hydrazid-, Hydrazon-, Ether-, Oxyd-, Cyanat-, Diazo-, Diazonium-, Sulfid-, Disulfid-, Sulfoxyd-, Sulfon-, Sulfonsäure-, Sulfinsäure-, Sulfat-, sulfensäure-, Aminid-, Imid-, Imin-, Imidat-, Nitron-, Hydroxylamin-, Oxim-, Hydroxaminsäure-, Thiohydroxyamidsäure-, Allen-, Ortho-Ester-, Sulfit-, Enamin-, Ynamin-, Harnstoff-, Pseudo-Harnstoff-, Semicarbazid-, Carbodiimid- und Carbamat-Gruppen; oder
- eine bioaktive Gruppe, ausgewählt aus Amiden, Estern, Aminosäuren, Peptiden, Proteinen, Enzymen, Polysacchariden, Nukleosiden, Nukleotiden, Oligonukleotiden, Radio-Isotopen, Carboranen, und Kombinationen derselben die folgenden Schritte umfassend:
i) Reagieren lassen einer Verbindung der Formel (I): 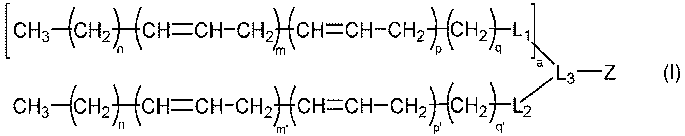 bei der a, **L₁, L₂, L₃, Z, n, n', m, m', p, p', q** und **q'** wie oben definiert sind; mit einer **R₅-L₄-SH** Verbindung, in der **L₄** und **R₅** wie oben definiert sind, in Gegenwart von mindestens einem Radikal-Initiator, mit;
ii) optional Reagieren lassen des Produkts der Reaktion (i) mit einem bioaktiven Molekül, ausgewählt aus Amiden, Estern, Aminosäuren, Peptiden, Proteinen, Enzymen, Polysacchariden, Nukleosiden, Nukleotiden, Oligonukleotiden, Radio-Isotopen, Carboranen, und Kombinationen derselben.

8. Pharmazeutische Zusammensetzung, umfassend eine Verbindung nach einem der Ansprüche **1** bis **4,** oder ein Liposom nach Anspruch **5,** oder ein Lipoplex nach Anspruch **6,** und einen physiologisch annehmbaren Träger.

9. Arzneimittel, umfassend eine Verbindung nach einem der Ansprüche **1** bis **4,** oder ein Liposom nach Anspruch **5,** oder ein Lipoplex nach Anspruch **6.**

10. Verwendung einer Verbindung nach einem der Ansprüche **1** bis **4,** oder eines Liposoms nach Anspruch **5,** oder eines Lipoplexes nach Anspruch **6** zur in vitro Vektorisierung.

11. Verwendung einer Verbindung nach einem der Ansprüche **1** bis **4,** oder eines Liposoms nach Anspruch **5,** oder eines Lipoplexes nach Anspruch **6** zur in vitro Transfektion.

12. Verbindung nach einem der Ansprüche **1** bis **4,** für deren Verwendung bei der in vivo Vektorisierung.

13. Lipoplex nach Anspruch **6,** für dessen Verwendung bei der in vivo Vektorisierung.

14. Verbindung nach einem der Ansprüche **1** bis **4,** für deren Verwendung bei der in vivo Transfektion, zur Abgabe von Prodrogen, Sensibilisatoren, oder eines Visualisierungsmittels für die Gentherapie, topische Behandlungen oder bakterizide Aktivitäten.

15. Liposom nach Anspruch **5,** für dessen Verwendung bei der Transfektion, zur Abgabe von Prodrogen, Sensibilisatoren, oder eines Visualisierungsmittels für die Gentherapie, topische Behandlungen oder bakterizide Aktivitäten.

16. Lipoplex nach Anspruch **6,** für dessen Verwendung bei der Transfektion, zur Abgabe von Prodrogen, Sensibilisatoren, oder eines Visualisierungsmittels für die Gentherapie, topische Behandlungen oder bakterizide Aktivitäten.

## Claims

1. Amphiphilic compound of general formula (II): 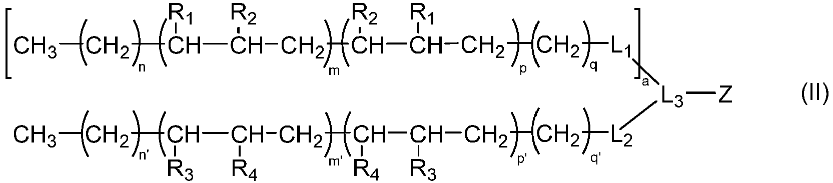 wherein:
**L₁** and **L₂** are each independently a linker, preferably the linker is chosen from a single bond and the alkyl, aryl, alkylaryl, arylalkyl, alkyloxy, alkyloxycarbonyl groups;
**L₃** is a linker, preferably the linker is chosen from the alkyl, alkylphosphoramidates, alkylthiophosphoramidates, alkylphosphate, alkylthiophosphate, alkylphosphonite, alkylphosphonate, alkylthiophosphonate, alkylphosphoramide, alkylthiophosphoramide, alkyloxy or amine groups;
**Z** is a polar functional group, said group being cationic, anionic, zwitterionic or neutral;
**a** is 1;
**n, n', q and q'** are each independently an integer from 1 to 15;
**m, m', p and p'** are each independently an integer from 0 to 4 with the condition that:
- at least one of m and p is different from 0;
- at least one of m' and p' is different from 0;
**R₁** and **R₂** are one a hydrogen and the other a thioether group of formula **-S-L₄-R₅** and **R₃** and **R₄** are one a hydrogen and the other a thioether group of formula **-S-L₄-R₅** such that:
**L₄** is a linker, preferably the linker is chosen from a single bond and the aminocarbonyl, acylamino, alkylaminocarbonyl, aminothiocarbonyl, alkyloxycarbonyl, alkyl, aryl, cycloalkyl, alkylaryl, arylalkyl, alkyloxy, polyethylene glycol (PEG), polypropylene glycol (PPG) groups, a peptide or a combination thereof; optionally interrupted or terminated by -O-, -S-, -SO₂- or a combination thereof; optionally further comprising a residue of a reactive group through which **L₄** is connected to **R₅;**
**R₅** is a hydrogen atom, or:
- polar group chosen from the group of organic salts of the ammonium, phosphonium, and imidazolium type and protonable neutral heterocycles;
- a reactive group chosen from the group comprising the group N₃, amino, alkylamino, COOH, amide, maleimide, alkyne, SH, OH, ester, activated ester, activated carboxylic acid, halo, nitro, nitrile, isonitrile, acrylamide, aldehyde, ketone , acetals, ketals, anhydride, thiocyanate, isothiocyanate, isocyanate, hydrazine, hydrazides, hydrazones, ethers, oxides, cyanates, diazo, diazonium, sulphides, disulphides, sulphoxides, sulphones, sulphonic acids, sulphinic acids, sulphates, sulphenic acids, amidines, imides, imines, imidates, nitrones, hydroxylamines, oximes, hydroxamic acids thiohydroxamic acids, allenes, ortho esters, sulphites, enamines, ynamines, ureas, pseudo-ureas, semicarbazides, carbodiimides, and carbamates; or
- a bioactive group selected from amides, esters, amino acids, peptides, proteins, enzymes, polysaccharides, nucleosides, nucleotides, oligonucleotides, radioisotopes, carboranes and combinations thereof. provided that when a is equal to 0 then Z is different from -S-CH2-CH2-OH and from -C(O)OMe.

2. Compound according to claim 1, of formula (IIa): 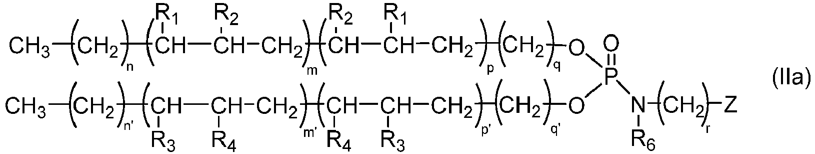
wherein **R₁, R₂, R₃, R₄, Z, n, n', m, m', p, p', q** and **q'** are such as defined in claim **1;**
**R₆** is a hydrogen or an alkyl;
**r** is an integer from 1 to 10.

3. Compound according to claim **1,** of formula (IIb): 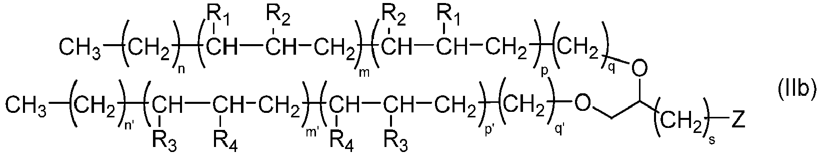
wherein **R₁, R₂, R₃, R₄, Z, n, n', m, m', p, p', q** and **q'** are such as defined in claim **1;**
s is an integer from 1 to 10.

4. Compound according to any of claims **1** to **3,** selected from the group comprising the compounds:
| | |
|---|---|
| Il-1a | 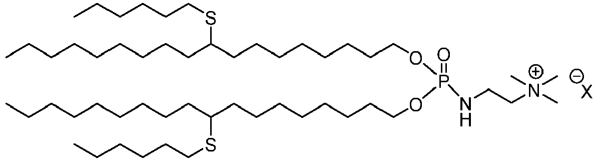 |
| II-1b | 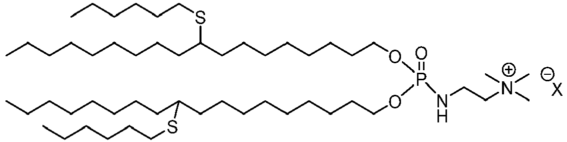 |
| II-1c | 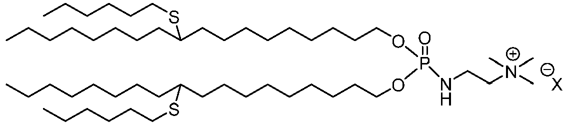 |
| II-2a | 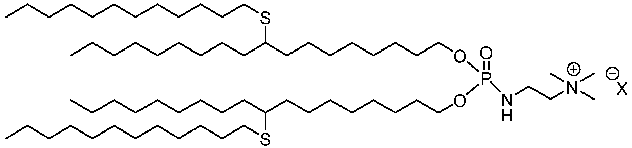 |
| II-2b | 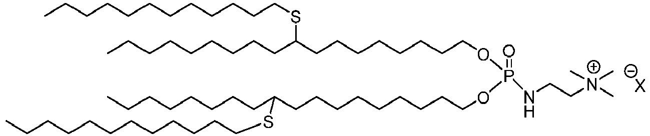 |
| II-2c | 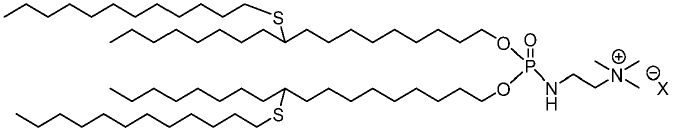 |
| II-3a | 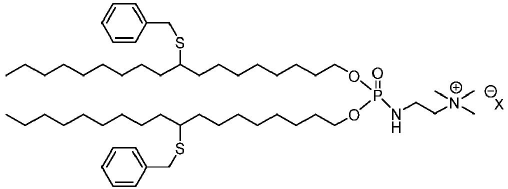 |
| II-3b | 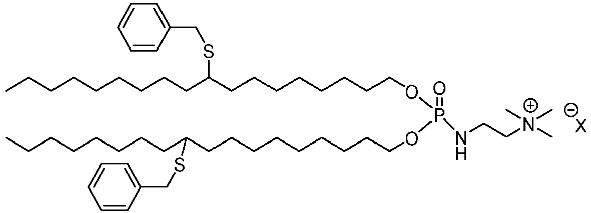 |
| II-3c | 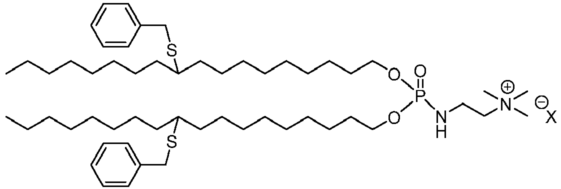 |
| II-4a | 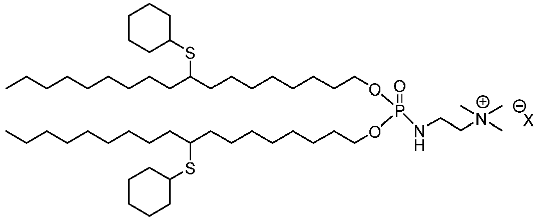 |
| II-4b | 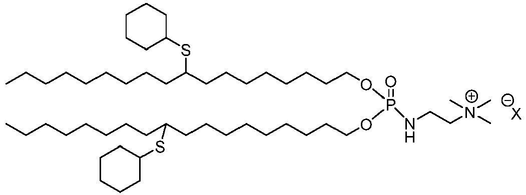 |
| II-4c | 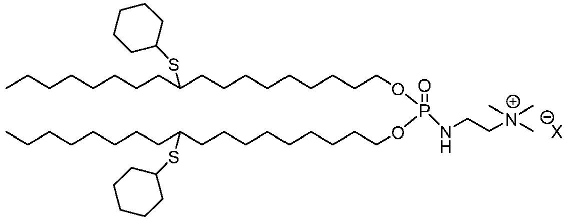 |
| II-5a | 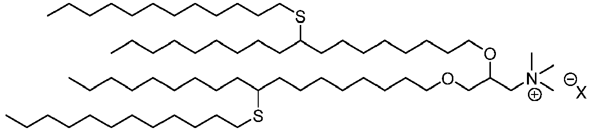 |
| II-5b | 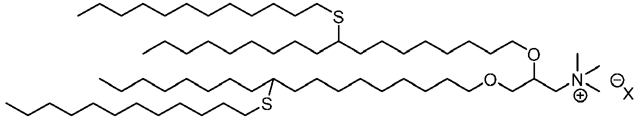 |
| II-5c | 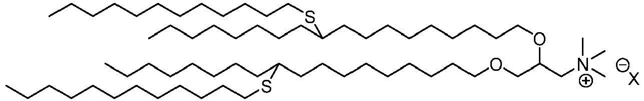 |
| II-6a | 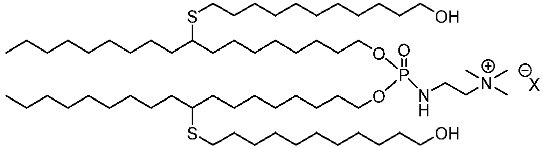 |
| II-6b | 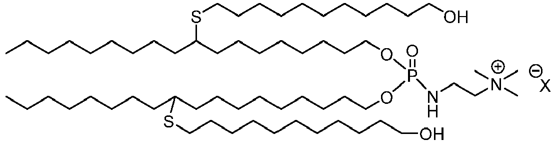 |
| II-6c | 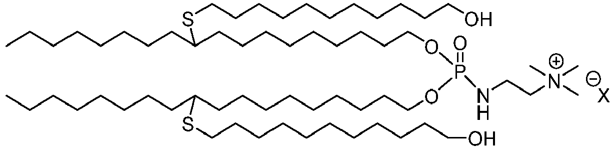 |
| II-7a | 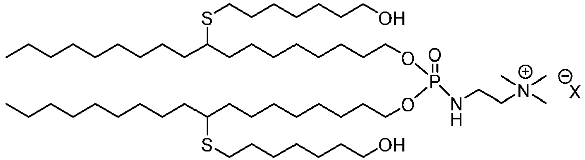 |
| II-7b | 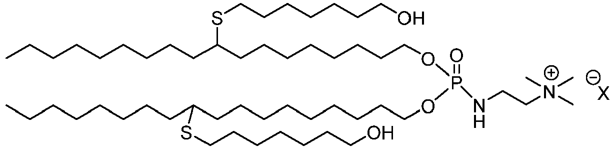 |
| II-7c | 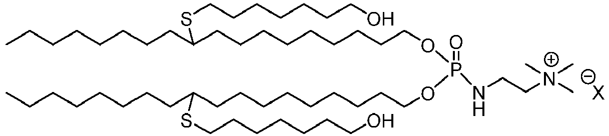 |
| II-8a | 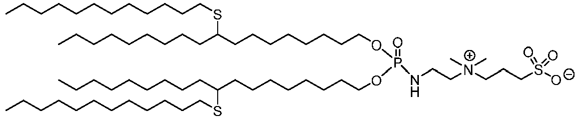 |
| II-8b | 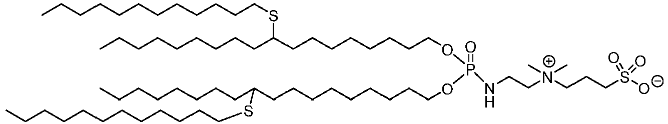 |
| II-8c | 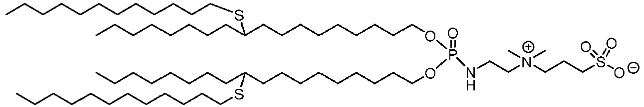 |
| II-9a | 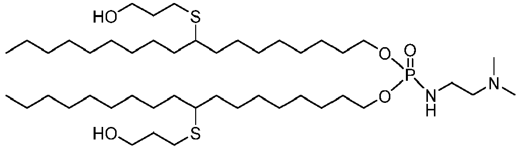 |
| II-9b | 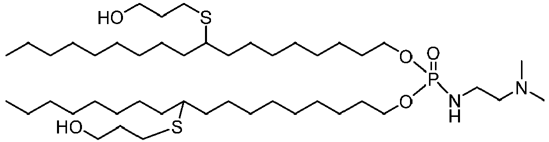 |
| II-9c | 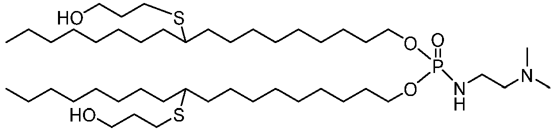 |
| II-10a | 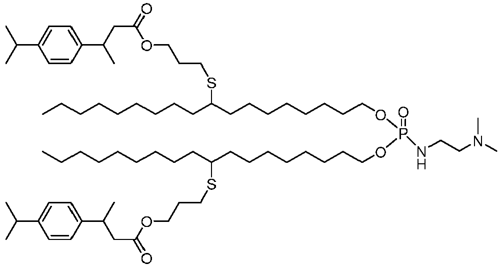 |
| II-10b | 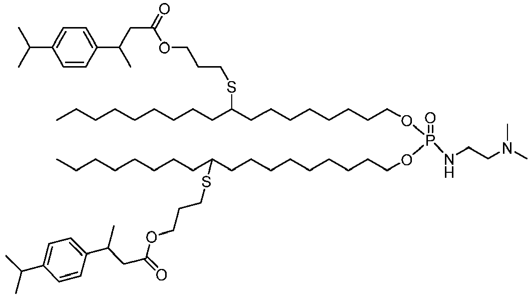 |
| II-10c | 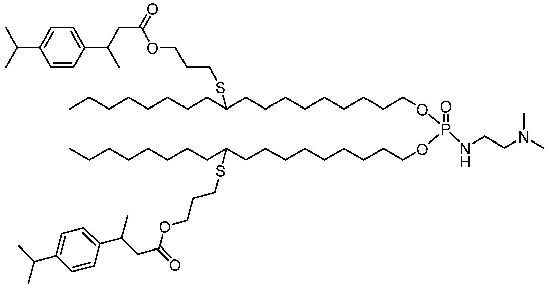 |
wherein X⁻ is a counterion.

5. Liposome comprising at least one of the compounds according to any of claims **1** to **4 characterised in that** it has a hexagonal phase.

6. Lipoplex comprising at least one of the compounds according to any of claims **1** to **4 characterised in that** it has a hexagonal phase.

7. Method for producing a compound of formula (II): 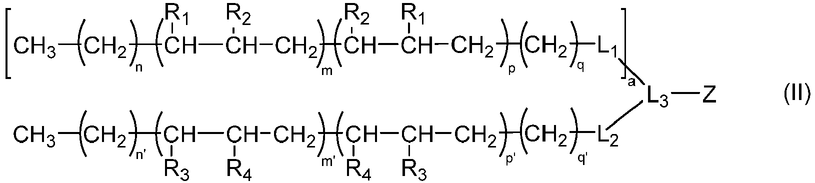 wherein:
**L₁** and **L₂** are each independently a linker, preferably the linker is chosen from a single bond and the alkyl, aryl, alkylaryl, arylalkyl, alkyloxy, alkyloxycarbonyl groups;
**L₃** is a linker, preferably the linker is chosen from the alkyl, alkylphosphoramidates, alkylthiophosphoramidates, alkylphosphate, alkylthiophosphate, alkylphosphonite, alkylphosphonate, alkylthiophosphonate, alkylphosphoramide, alkylthiophosphoramide, alkyloxy or amine groups;
**Z** is a polar functional group, said group being cationic, anionic, zwitterionic or neutral;
**a** is 1;
**n, n', q and q'** are each independently an integer from 1 to 15;
**m, m', p and p'** are each independently an integer from 0 to 4 with the condition that:
- at least one of m and p is different from 0;
- at least one of m' and p' is different from 0;
**R₁** and **R₂** are one a hydrogen and the other a thioether group of formula **-S-L₄-R₅** and **R₃** and **R₄** are one a hydrogen and the other a thioether group of formula **-S-L₄-R₅** wherein:
**L₄** is a linker, preferably the linker is chosen from a single bond and the aminocarbonyl, acylamino, alkylaminocarbonyl, aminothiocarbonyl, alkyloxycarbonyl, alkyl, aryl, cycloalkyl, alkylaryl, arylalkyl, alkyloxy, polyethylene glycol (PEG), polypropylene glycol (PPG) groups, a peptide or a combination thereof; optionally interrupted or terminated by -O-, -S-, -SO₂- or a combination thereof; optionally further comprising a residue of a reactive group through which **L₄** is connected to **R₅;**
**R₅** is a hydrogen atom, or:
- polar group chosen from the group of organic salts of the ammonium, phosphonium, and imidazolium type and protonable neutral heterocycles;
- a reactive group chosen from the group comprising the N3, amino, alkylamino, COOH, amide, maleimide, alkyne, SH, OH, ester, activated ester, activated carboxylic acid, halo, nitro, nitrile, isonitrile, acrylamide, aldehyde, ketone, acetals, ketals, anhydride, thiocyanate, isothiocyanate, isocyanate, hydrazine, hydrazides, hydrazones, ethers, oxides, cyanates, diazo, diazonium, sulphides, disulphides, sulphoxides, sulphones, sulphonic acids, sulphinic acids, sulphates, sulphenic acids, amidines, imides, imines, imidates, nitrones, hydroxylamines, oximes, hydroxamic acids thiohydroxamic acids, allenes, ortho esters, sulphites, enamines, ynamines, ureas, pseudo-ureas, semicarbazides, carbodiimides, carbamates group; or
- a bioactive group chosen from amides, esters, amino acids, peptides, proteins, enzymes, polysaccharides, nucleosides, nucleotides, oligonucleotides, radioisotopes, the carboranes, and combinations thereof;
comprising the following steps:
i) reacting a compound of formula (I): 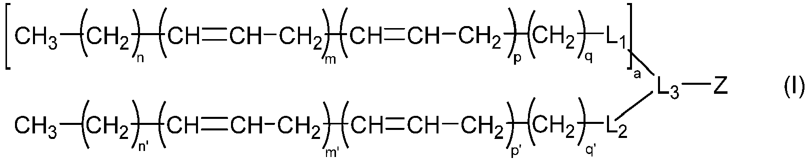 wherein **a, L₁, L₂, L₃, Z, n, n', m, m', p, p', q** and **q'** are such as defined hereinabove;
with a compound **R₅-L₄-SH** wherein **L₄** and **R₅** are such as defined hereinabove, in the presence of at least one radical initiator with;
ii) optionally, reacting the product of the reaction (i) with a bioactive molecule, chosen from amides, esters, amino acids, peptides, proteins, enzymes, polysaccharides, nucleosides, nucleotides, oligonucleotides, radioisotopes, and carboranes, and combinations thereof.

8. Pharmaceutical composition comprising a compound according to any of claims **1** to **4** or a liposome according to claim **5** or a lipoplex according to claim **6** and a physiologically acceptable vehicle.

9. Medicament comprising a compound according to any of claims **1** to **4** or a liposome according to claim **5** or a lipoplex according to claim **6.**

10. Use of a compound according to any of claims **1** to **4** or of a liposome according to claim **5** or of a lipoplex according to claim **6** for vectorisation in vitro.

11. Use of a compound according to any of claims **1** to **4** or of a liposome according to claim **5** or of a lipoplex according to claim **6** for transfection in vitro.

12. Compound according to any of claims 1 to 4 for the use thereof for vectorisation in vivo.

13. Lipoplex according to claim 6 for the use thereof for vectorisation in vivo.

14. Compound according to any of claims 1 to 4 for the use thereof in transfection in vivo, for the delivery of prodrugs, sensitisers or imaging agent, for gene therapy, topical treatments or bactericidal activities.

15. Liposome according to claim 5 for the use thereof in transfection, for the delivery of prodrugs, sensitisers or imaging agent, for gene therapy, topical treatments or bactericidal activities.

16. Lipoplex according to claim 6 for the use thereof in transfection, for the delivery of prodrugs, sensitisers or imaging agent, for gene therapy, topical treatments or bactericidal activities.
